# EUROPEAN PATENT APPLICATION

(11) **EP 4 470 461 A1**
(43) Date of publication of application: **04.12.2024**
(21) Application number: 23803685.9
(22) Date of filing: 23.03.2023
(51) Int. Cl.: A61B 5/11, A61B 5/00, B25J 9/00, B25J 13/08, B25J 9/16

(54) **ELECTRONIC DEVICE AND WEARABLE DEVICE FOR PROVIDING INFORMATION ON EXERCISE CAPACITY OF USER, AND OPERATION METHODS THEREOF**

(30) Priority: 09.05.2022 KR 20220056747; 30.09.2022 KR 20220125002
(71) Applicant: Samsung Electronics Co., Ltd, Suwon-si, Gyeonggi-do 16677 (KR)
(72) Inventor: KIM, Dongwoo, Suwon-si Gyeonggi-do 16677 (KR); HYEON, Jaehun, Suwon-si Gyeonggi-do 16677 (KR); LEE, Kiwan, Suwon-si Gyeonggi-do 16677 (KR); LEE, Hwangjae, Suwon-si Gyeonggi-do 16677 (KR); LEE, Kwanghyung, Seoul 06294 (KR); JEON, Myeonghee, Seoul 06294 (KR); JEONG, Yuchang, Seoul 06294 (KR); HA, Jiwon, Seoul 06294 (KR)
(74) Representative: Gulde & Partner
(86) International application number: PCT/KR2023/003838
(87) International publication number: WO 2023/219257

(57) **Abstract**

Disclosed are an electronic device and a wearable device for providing information on the exercise capacity of a user, and an operation method of the electronic device performing same. The electronic device comprises: a communication module for receiving, from the wearable device, sensor data including movement information of a user wearing the wearable device; a processor for estimating the exercise capacity of the user on the basis of the sensor data and generating exercise capacity information about the estimated exercise capacity; and a display module for outputting the exercise capacity information. The sensor data includes exercise information about movements performed by the user in accordance with one or more exercise programs presented to the user when the wearable device is in an exercise capacity evaluation mode. The processor determines the exercise capacity information on the basis of the exercise information and evaluation criteria information corresponding to the one or more exercise programs.

## Description

### TECHNICAL FIELD

The following embodiments relate to an electronic device and a wearable device for providing exercise ability information of a user and operation methods thereof.

### BACKGROUND ART

Typically, a walking assistance device may be equipment or a device for assisting a patient who is not able to walk by themselves due to various diseases or accidents to perform walking exercise for rehabilitation. As the number of aging individuals increases, a growing number of people experience inconvenience in walking or have difficulty walking normally due to malfunctioning joint issues, and there is increasing interest in walking assistance devices. A walking assistance device may be worn on a body of a user to assist the user with walking and/or exercise by providing the necessary muscular strength and/or to induce the user to walk in a normal walking pattern.

### DISCLOSURE OF THE INVENTION

### TECHNICAL SOLUTIONS

According to an embodiment, an electronic device may include a communication module configured to receive sensor data including motion information of a user who wears a wearable device from the wearable device, a processor configured to estimate an exercise ability of the user based on the sensor data and generate exercise ability information on the estimated exercise ability, and a display module configured to output the exercise ability information. The sensor data may include exercise information on a motion performed by the user according to at least one exercise program provided to the user when the wearable device operates in an exercise ability assessment mode. The processor may be further configured to determine the exercise ability information based on the exercise information and an assessment reference information corresponding to the at least one program.

According to an embodiment, a wearable device worn on a body of a user may include a support frame configured to support the body of the user when the wearable device is worn on the body of the user, a sensor module configured to obtain sensor data including motion information of the user, a driving module configured to generate torque applied to a body part of the user through the support frame, a communication module configured to transmit the sensor data to an electronic device and receive a control signal from the electronic device, and a processor configured to control the communication module and the sensor module. The sensor data may include exercise information on a motion performed by the user according to at least one exercise program provided to the user when the wearable device operates in an exercise ability assessment mode. The processor may be further configured to control the communication module to transmit the sensor data to the electronic device to cause the electronic device to estimate an exercise ability of the user based on the sensor data, and determine exercise ability information of the user based on the exercise information and assessment reference information corresponding to the at least one exercise program.

According to an embodiment, a method of operating an electronic device may include, in response to the user input, transmitting a control signal to measure the exercise ability information to the wearable device, in response to the transmission of the control signal, when the wearable device operates in an exercise ability assessment mode, receiving, from the wearable device, sensor data including exercise information on a motion performed by the user according to at least one exercise program provided to the user, and determining the exercise ability information of the user based on the sensor data. The determining of the exercise ability information may include determining the exercise ability information based on the exercise information and assessment reference information corresponding to the at least one exercise program.

### BRIEF DESCRIPTION OF DRAWINGS

FIG. 1 illustrates an overview of a wearable device worn on a body of a user according to an embodiment.
FIG. 2 is a diagram illustrating a management system including a wearable device and an electronic device according to an embodiment.
FIG. 3 is a front view of a wearable device according to an embodiment.
FIG. 4 is a side view of a wearable device according to an embodiment.
FIGS. 5A and 5B are diagrams illustrating a configuration of a control system of a wearable device according to an embodiment.
FIG. 6 is a diagram illustrating an interaction between a wearable device and an electronic device according to an embodiment.
FIG. 7 is a diagram illustrating a configuration of an electronic device according to an embodiment.
FIG. 8 is a diagram illustrating an operation method of an electronic device for determining exercise ability information of a user according to an embodiment.
FIG. 9 is a diagram illustrating an interaction among an electronic device, a wearable device, and another wearable device according to an embodiment.
FIG. 10 is a diagram illustrating a process of determining exercise ability information of a user when the user wears a wearable device and performs a standing knee-to-chest exercise, according to an embodiment.
FIG. 11 is a diagram illustrating a process of determining exercise ability information of a user when the user wears a wearable device and performs a knee-up exercise, according to an embodiment.
FIG. 12 is a diagram illustrating a process of measuring a walking index of a user using a wearable device according to an embodiment.

### BEST MODE FOR CARRYING OUT THE INVENTION

The following detailed structural or functional description is provided as an example only and various alterations and modifications may be made to the embodiments. Here, the embodiments are not construed as limited to the disclosure and should be understood to include all changes, equivalents, and replacements within the idea and the technical scope of the disclosure.

The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and a repeated description related thereto will be omitted.

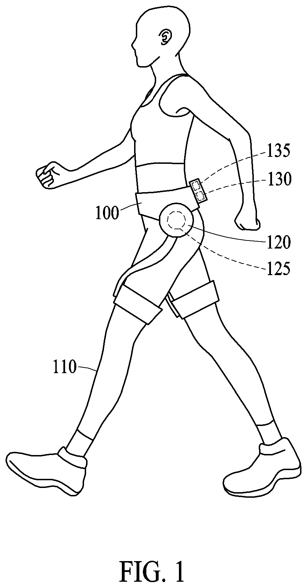

FIG. 1 is a diagram illustrating an overview of a wearable device worn on a user's body, according to an embodiment.

Referring to FIG. 1, in an embodiment, a wearable device 100 may be a device worn on a body of a user 110 to assist the user 110 in walking, exercising, and/or working. In an embodiment, the wearable device 100 may also be used to measure a physical ability (e.g., a walking ability, and an exercise ability) of the user 110. In embodiments, the term "wearable device" may be replaced with "wearable robot," "walking assistance device," or "exercise assistance device". The user 110 may be a human or an animal, but is not limited thereto. The wearable device 100 may be worn on the body (e.g., a lower body (legs, ankles, knees, etc.) or a waist) of the user 110 and may apply an external force, such as an assistance force and/or a resistance force, to a body motion of the user 110. The assistance force may be a force assisting the body motion of the user 110, which is applied in the same direction as a direction of the body motion of the user 110. The resistance force may be a force impeding the body motion of the user 110, which is applied in an opposite direction to the direction of the body motion of the user 110. The term "resistance force" may also be referred to as an "exercise load".

In an embodiment, when the wearable device 100 operates in the walking assistance mode for assisting the user 110 in walking, the wearable device 100 may assist the walking of the user 110 by applying an assistance force generated through a driving module 120 of the wearable device 100 to the body of the user 110. The wearable device 100 may expand a walking ability of the user 110 by allowing the user 110 to walk independently or walk for a long time by providing a force needed for the walking of the user 110. The wearable device 100 may also improve the walking of a user having an abnormal walking habit or posture.

In an embodiment, when the wearable device 100 operates in an exercise assistance mode for enhancing the exercise effect of the user 110, the wearable device 100 may hinder a body motion of the user 110 or may provide resistance to a body motion of the user 110 by applying a resistance force generated by the driving module 120 to the body of the user 110. When the wearable device 100 is a hip-type wearable device that is worn on the waist (or pelvis) and legs (e.g., thighs) of the user 110, the wearable device 100 may provide an exercise load to a body motion of the user 110 while being worn on the legs, thereby enhancing the exercise effect on the legs of the user 110. In an embodiment, in the exercise assistance mode, the wearable device 100 may apply an assistance force to the body of the user 110 to assist a body motion of the user 110. In an embodiment, in the exercise assistance mode, the wearable device 100 may provide a combination of an assistance force and a resistance force for each exercise session or time interval, for example, may provide an assistance force in some exercise sessions, and may provide a resistance force in other exercise sessions.

In an embodiment, when the wearable device 100 operates in an exercise ability assessment mode for measuring an exercise ability of the user 110, the wearable device 100 may measure motion information of the user using sensors (e.g., the angle sensor 125 and the IMU 135) provided in the wearable device 100 while the user walks or performs an exercise, and may assess the exercise ability of the user based on the measured motion information. The sensors may be used to obtain the motion information of each component of the wearable device 100.

In embodiments of the present disclosure, for convenience of description, the wearable device 100 is described as an example of a hip-type wearable device, as illustrated in FIG. 1, but the embodiments are not limited thereto. As described above, the wearable device 100 may be worn on another body part (e.g., the upper arms, lower arms, hands, calves, and feet) other than the waist and legs (particularly the thighs), and the shape and configuration of the wearable device 100 may vary depending on the body part on which the wearable device 100 is worn.

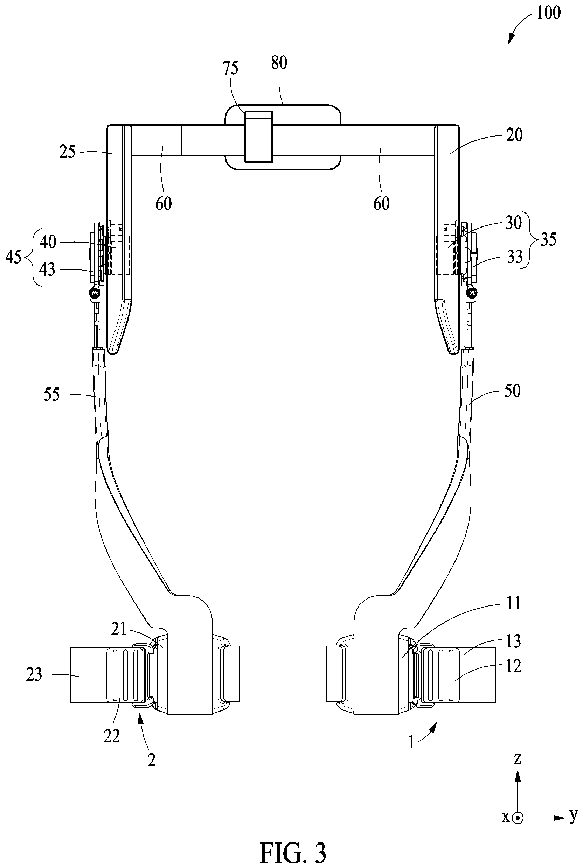

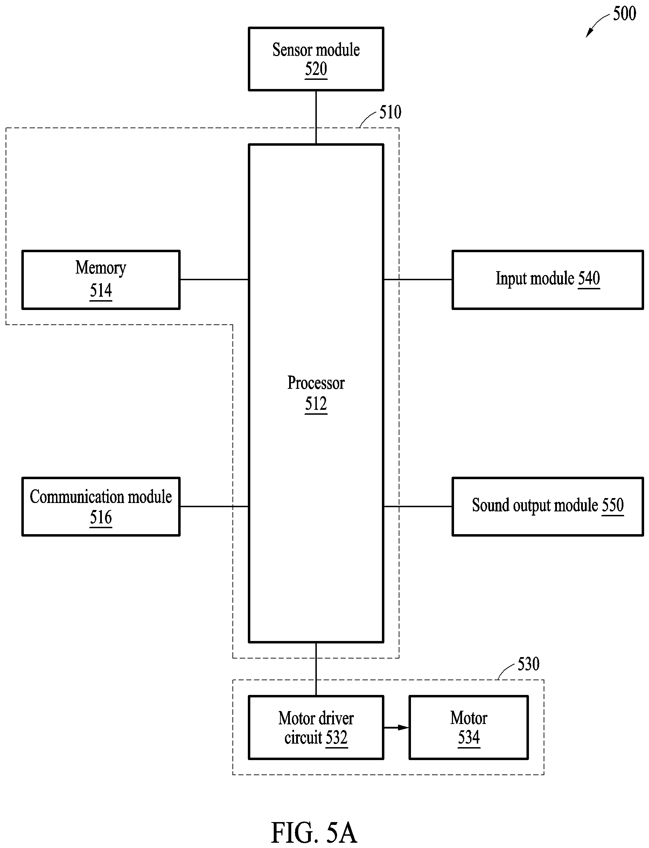

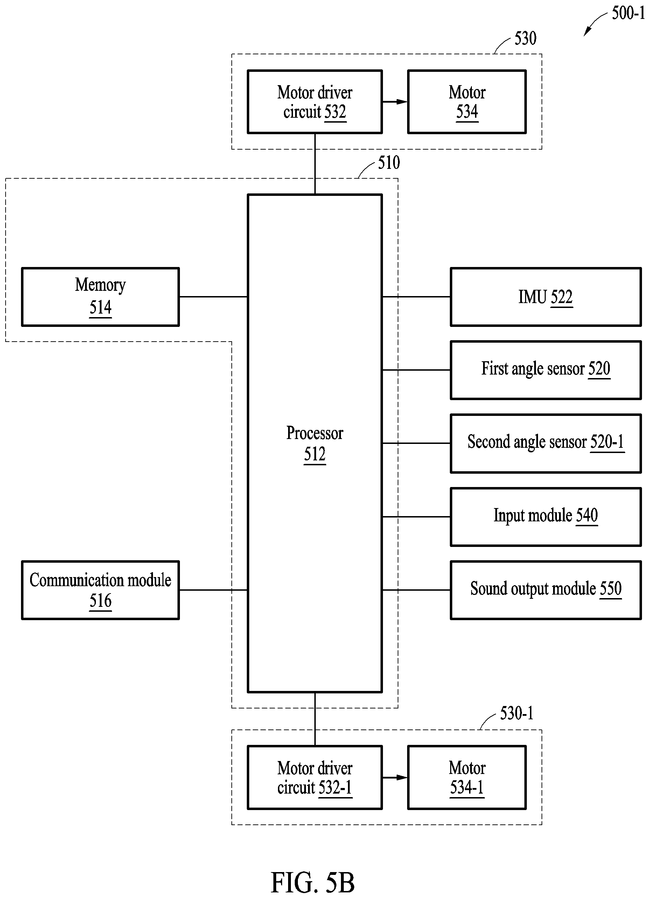

According to an embodiment, the wearable device 100 may include a support frame (e.g., leg support frames 50 and 55 and waist support frames 20 and 25 of FIG. 3) configured to support the body of the user 110 when the wearable device 100 is worn on the body of the user 110, a sensor module (e.g., a sensor module 520 of FIG. 5A) configured to obtain sensor data including motion information a motion of a component of the wearable device 100 and/or a body motion (e.g., a leg motion or an upper body motion) of the user, the driving module 120 (e.g., driving modules 35 and 45 of FIG. 3) configured to generate an external force applied to a leg of the user, and a control module 130 (e.g., a control module 510 of FIGS. 5A and 5B) configured to control the wearable device 100.

The sensor module may include the angle sensor 125 and the IMU 135. The angle sensor 125 may measure a hip joint angle value of the user 110. The angle sensor 125 may include, for example, an encoder and/or a hall sensor. In an embodiment, the angle sensors 125 may be positioned near the left hip joint and the right hip joint and may measure a hip joint angle value of the left hip joint of the user 110 and a hip joint angle value of the right hip joint of the user 110, respectively. The hip joint angle value of the left hip joint of the user 110 may correspond to an angle of the left leg of the user 110, and the hip joint angle value of the right hip joint of the user 110 may correspond to an angle of the right leg of the user 110. In an embodiment, the angle sensor 125 may obtain a motion value of a leg support frame of the wearable device 100.

The IMU 135 may measure a change in acceleration and rotation velocity according to the motion of the user 110. The IMU 135 may include an acceleration sensor and/or an angular velocity sensor. The IMU 135 may measure, for example, a motion value of the upper body of the user 110. The motion value of the upper body may correspond to a motion value of a waist support frame of the wearable device 100.

In an embodiment, the control module 130 and the IMU 135 may be disposed in a housing (e.g., a housing 80 of FIG. 3) of the wearable device 100. The housing may be formed or attached to the outside of the support frame of the wearable device 100, and may be disposed behind the waist when the user 110 wears the wearable device 100, however, the embodiments are not limited thereto.

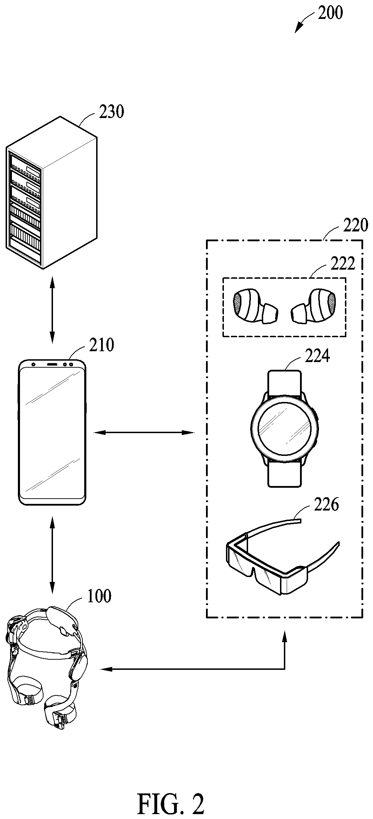

When the user 110 intends to use the wearable device 100, the user 110 may generally input a control command to drive the wearable device 100 while wearing the wearable device 100. For example, the user 110 may drive the wearable device 100 by pressing a separate button included by the wearable device 100 or inputting a driving command to an application of an electronic device (e.g., an electronic device 210 of FIG. 2) interoperating with the wearable device 100. When the user 110 desires to stop using the wearable device 100 while using the wearable device 100, the user 110 may input a control command to stop the driving of the wearable device 100 and may turn off the power of the wearable device 100.

In an embodiment, the user 110 may input a user input for measuring exercise ability information to an electronic device (e.g., the electronic device 210 of FIG. 2) interoperating with the wearable device 100 to measure the exercise ability of the user 110. In response to the user input, the electronic device may transmit a control signal to measure the exercise ability information to the wearable device 100. The wearable device 100 that receives the control signal may obtain sensor data including the motion information of the user 110 through the sensor module.

In an embodiment, in response to the user input for measuring the exercise ability, the electronic device may output a user interface (UI) screen to a display module of the electronic device to guide the exercise ability measurement. The user 110 may receive a recommendation of at least one exercise program to measure the exercise ability information of the user 110. The sensor data may include exercise information on a motion performed by the user according to the at least one exercise program provided to the user. A processor included in the electronic device may determine the exercise ability information of the user 110 based on the exercise information and assessment reference information corresponding to the at least one exercise program. For example, the exercise ability information may include information on at least one of the flexibility, symmetry of flexibility, physical strength, symmetry of strength, core stability, or cardiorespiratory endurance of the user 110.

According to an embodiment, the processor of the electronic device may assess an exercise posture of the user 110 for a reference exercise posture defined by the at least one exercise program provided to the user 110 based on the sensor data and may determine the exercise ability information based on a result of the assessment. The reference exercise posture defined by the at least one exercise program may vary depending on the exercise program. For example, the reference exercise posture may be a standing knee-to-chest motion or a knee-up motion, but the example is not limited thereto.

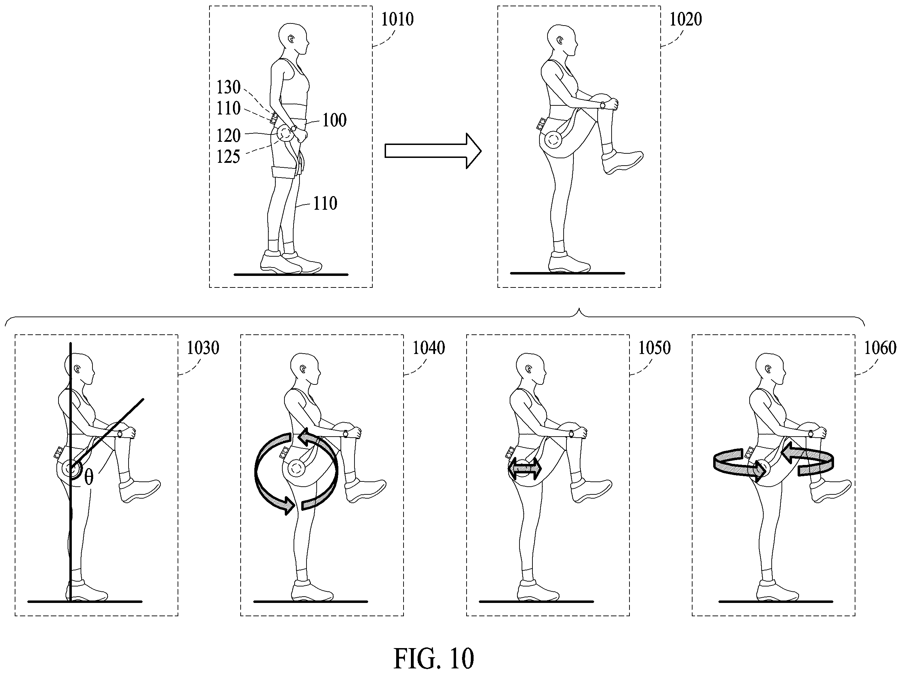

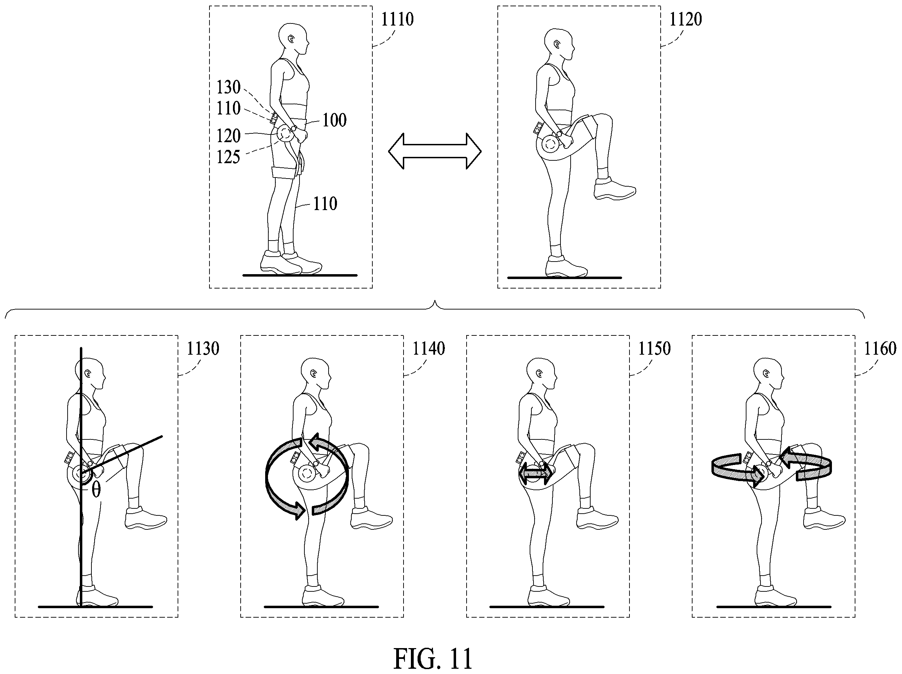

The reference exercise posture may include a plurality of posture items. The processor of the electronic device may determine an assessment result of each of the plurality of posture items based on the assessment criteria defined for each of the plurality of posture items, based on the sensor data. For example, the plurality of posture items may include at least one of the number of motions, the motion velocity, a hip joint angle, or the body stability during the exercise, but the example is not limited thereto. A detailed description thereof is provided with reference to FIGS. 10 and 11.

According to an embodiment, the electronic device may determine the exercise ability information more accurately based on the assessment reference information corresponding to each exercise program and hip joint angle values of both legs of the user 110 obtained by the sensor module included in the wearable device 100, and/or an acceleration value and an angular velocity value according to the motion of the user and may provide the determined exercise ability information to the user through an application.

In an embodiment, when a predefined condition is satisfied while the user 110 moves according to the exercise ability assessment mode, the magnitude of the resistance force applied to the user 110 by the wearable device 100 may change. For example, when a preset time has arrived, or an exercise session has changed, the wearable device 100 may increase the magnitude of the resistance force applied to the user 110. Depending on the embodiments, the user 110 may adjust the magnitude of the resistance force through an electronic device linked with the wearable device 100.

Based on the determined exercise ability information, the user 110 may set the magnitude of the resistance force appropriate for their exercise level and a condition for changing the magnitude of the resistance force through the electronic device. For example, the user 110 may more accurately assess their exercise posture using the electronic device and the wearable device 100 and may improve the exercise level by correcting the exercise posture based on the assessed exercise posture.

FIG. 2 is a diagram illustrating a management system including a wearable device and an electronic device according to an embodiment.

Referring to FIG. 2, a management system 200 may include the wearable device 100 for assisting a body motion of a user, an electronic device 210, other wearable devices 220, and a server 230. In an embodiment, at least one (e.g., the other wearable device 220 or the server 230) of these devices may be omitted from the management system 200, or one or more other devices (e.g., an exclusive controller device for the wearable device 100) may be added to the management system 200.

In an embodiment, the wearable device 100 may be worn on a body of the user in the walking assistance mode to assist a motion of the user. For example, the wearable device 100 may be worn on the legs of the user to help the user in walking by generating an assistance force for assisting a leg motion of the user. In an embodiment, the wearable device 100 may generate and apply a resistance force to the body of the user to hinder a body motion of the user to enhance the exercise effect of the user in the exercise assistance mode.

In an embodiment, the wearable device 100 may be used to measure the exercise ability of the user by linking with the electronic device 210. The wearable device 100 may operate in the exercise ability assessment mode, which is a mode for measuring the exercise ability of the user under the control of the electronic device 210, and may transmit, to the electronic device 210, the sensor data obtained by the motion of the user in the exercise ability assessment mode. The electronic device 210 may estimate the exercise ability of the user by analyzing the sensor data received from the wearable device 100.

The electronic device 210 may communicate with the wearable device 100 and may remotely control the wearable device 100 or may provide state information (e.g., remaining battery power) of the wearable device 100 to the user. The electronic device 210 may receive, from the wearable device 100, the sensor data obtained by a sensor in the wearable device 100 and may determine a physical state or the exercise ability of the user based on the received sensor data. In an embodiment, the electronic device 210 may execute a program (e.g., an application) to control the wearable device 100, and the user may adjust a setting value (e.g., the magnitude of torque or a volume of audio) or an operation of the wearable device 100 through the program. The electronic device 210 may be a device in various forms. The electronic device 210 may include, for example, a portable communication device (e.g., a smartphone), a computer device, a portable multimedia device, or a home appliance, but is not limited thereto.

In an embodiment, the electronic device 210 may be connected to the server 230 using short-range wireless communication or cellular communication. The server 230 may receive user information (e.g., name, age, gender) on a user using the wearable device 100 and/or exercise ability information on the user from the electronic device 210 and may store and manage the received user information and/or the exercise ability information. The server 230 may provide, to the electronic device 210, various exercise programs or exercise ability measurement programs to be provided to the user by the wearable device 100.

According to an embodiment, the wearable device 100 and/or the electronic device 210 may be connected to the other wearable devices 220. The other wearable devices 220 may include, for example, wireless earphones 222, a smartwatch 224, or smart glasses 226, but are not limited thereto. In an embodiment, the exercise ability information generated by the electronic device 210 and/or the state information of the wearable device 100 may be transmitted to the other wearable devices 220 and may be provided to the user through the other wearable devices 220. In an embodiment, the wearable device 100, the electronic device 210, and the other wearable device 220 may be connected to each other via wireless communication (e.g., Bluetooth communication).

In an embodiment, after the electronic device 210 determines the exercise ability information on the user based on the sensor data obtained by the wearable device 100, the electronic device 210 may transmit the exercise ability information to the other wearable devices 220. The user may receive a guide for the exercise ability assessment of the user through a display module or a sound output module of the other wearable devices 220 and may receive the exercise ability information determined by the electronic device 210.

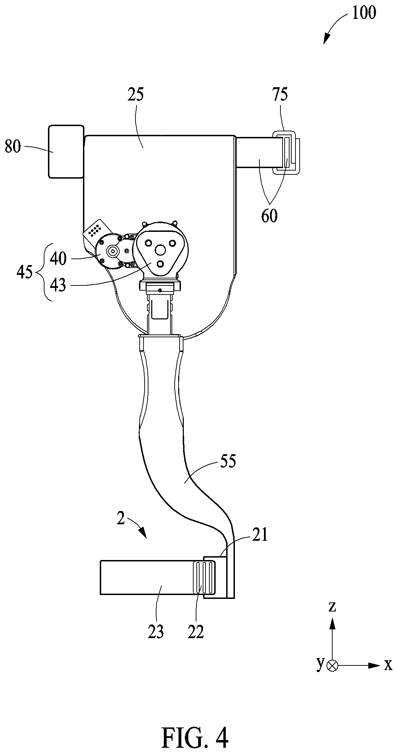

FIG. 3 is a front view of a wearable device according to an embodiment, and FIG. 4 is a side view of a wearable device according to an embodiment.

Referring to FIGS. 3 and 4, the wearable device 100 worn on the body of a user in an embodiment may include a housing 80, waist support frames 20 and 25, driving modules 35 and 45, leg support frames 50 and 55, thigh fasteners 1 and 2, and a waist fastener. The waist fastener may include a belt 60 and an auxiliary belt 75. In an embodiment, at least one (e.g., the auxiliary belt 75) of the components described above may be omitted from the wearable device 100 or one or more other components may be added to the wearable device 100.

In an embodiment, a control module (not shown) (e.g., the control module 130 of FIG. 1 and the control module 510 of FIGS. 5A and 5B), an IMU (not shown) (e.g., the IMU 135 of FIG. 1 and an IMU 522 of FIG. 5B), a communication module (not shown) (e.g., a communication module 516 of FIGS. 5A and 5B), and a battery (not shown) may be disposed inside the housing 80. The housing 80 may protect the control module, the IMU, the communication module, and the battery. For example, the housing 80 may be disposed on the back or rear side of the waist of a user based on a state in which the wearable device 100 is worn on the body of the user. The control module may generate a control signal for controlling an operation of the wearable device 100. The control module may include a control circuit including a memory and a processor configured to control actuators 30 and 40 of the driving modules 35 and 45. The control module may further include a power supply module (not shown) configured to supply power from the battery to each component of the wearable device 100.

In an embodiment, the wearable device 100 may include a sensor module (not shown) (e.g., the sensor module 520 of FIG. 5A) configured to obtain sensor data from at least one sensor. The sensor module may obtain sensor data that changes according to the motion of a user. In an embodiment, the sensor module may obtain sensor data including motion information of the user and/or motion information of the components of the wearable device 100. The sensor module may include, for example, an IMU (e.g., the IMU 135 of FIG. 1 or the IMU 522 of FIG. 5B) configured to measure a motion value of the upper body of the user or a motion value of the waist support frame 20 or 25 and an angle sensor (e.g., the angle sensor 125 of FIG. 1, a first angle sensor 520 and a second angle sensor 520-1 of FIG. 5B) configured to measure a hip joint angle value of the user or a motion value of the leg support frame 50 or 55. However, the example is not limited thereto. For example, the sensor module may further include at least one of a position sensor, a temperature sensor, a biosignal sensor, and a proximity sensor.

In an embodiment, the waist support frames 20 and 25 may support a body part of the user when the wearable device 100 is worn on the body of the user. The waist support frames 20 and 25 may contact at least a portion of an outer surface of the user. The waist support frames 20 and 25 may be bent in a shape corresponding to a portion in contact with the body of the user. For example, the waist support frames 20 and 25 may have a shape surrounding the outer surface of the waist (or pelvis) of the user and may support the waist or pelvis of the user. The waist support frames 20 and 25 may include the first waist support frame 25 configured to support a right side of the waist of the user, and the second waist support frame 20 configured to support a left side of the waist of the user. The waist support frames 20 and 25 may be connected to the housing 80.

The waist fastener may be connected to the waist support frames 20 and 25 and may fasten the waist support frames 20 and 25 to the waist of the user. The waist fastener may include, for example, a pair of belts 60 and the auxiliary belt 75. The auxiliary belt 75 may be connected to one of the pair of belts 60.

In an embodiment, the pair of belts 60 may be connected to the waist support frames 20 and 25. In a state before the user wears the wearable device 100, the pair of belts 60 may maintain a shape extending in a front direction (e.g., the +x direction) and may not hinder the user from entering inside the pair of waist support frames 20 and 25. When the user enters inside the pair of waist support frames 20 and 25, the pair of belts 60 may be deformed and may surround a front portion of the user. The waist support frames 20 and 25 and the pair of belts 60 may entirely surround the circumference of the waist of the user. In an embodiment, the auxiliary belt 75 may fasten the pair of belts 60 to each other while the pair of belts 60 overlaps with each other. For example, one of the pair of belts 60 may wrap the other belt together with the auxiliary belt 75.

The driving modules 35 and 45 may generate an external force (or torque) to be applied to the body of the user based on a control signal generated by the control module. The driving modules 35 and 45 may generate an external force to be applied to the user's legs under the control of the control module. In an embodiment, the driving modules 35 and 45 may include the first driving module 45 disposed at a position corresponding to a right hip joint of the user and the second driving module 35 disposed at a position corresponding to a left hip joint of the user. The first driving module 45 may include a first actuator 40 and a first joint member 43, and the second driving module 35 may include a second actuator 30 and a second joint member 33. The first actuator 40 may provide power to be transmitted to the first joint member 43 and the second actuator 30 may provide power to be transmitted to the second joint member 33. The first actuator 40 and the second actuator 30 may each include a motor configured to generate power (or torque) by receiving power from a battery. When the motor receives power and is driven, the motor may provide a force (an assistance force) for assisting a body motion of the user or a force (a resistance force) for hindering a body motion of the user. In an embodiment, the control module may adjust the magnitude or direction of a force generated by the motor by adjusting a voltage or a current supplied to the motor.

In an embodiment, the first joint member 43 and the second joint member 33 may receive power from the first actuator 40 and the second actuator 30, respectively, and may apply an external force to the body of the user based on the received power. The first joint member 43 and the second joint member 33 may be disposed at positions corresponding to joint portions of the user, respectively. The first joint member 43 and the second joint member 33 may be disposed on one side of the waist support frames 25 and 20, respectively. One side of the first joint member 43 may be connected to the first actuator 40, and the other side of the first joint member 43 may be connected to the first leg support frame 55. The first joint member 43 may rotate by the power received from the first actuator 40. An encoder or a hall sensor that may operate as an angle sensor configured to measure a rotation angle (corresponding to a joint angle of the user) of the first joint member 43 may be disposed on one side of the first joint member 43. One side of the second joint member 33 may be connected to the second actuator 30, and the other side of the second joint member 33 may be connected to the second leg support frame 50. The second joint member 33 may rotate by the power received from the second actuator 30. An encoder or a hall sensor that may operate as an angle sensor configured to measure a rotation angle of the second joint member 33 may be disposed on one side of the second joint member 33.

In an embodiment, the first actuator 40 may be disposed in a lateral direction of the first joint member 43, and the second actuator 30 may be disposed in a lateral direction of the second joint member 33. A rotation axis of the first actuator 40 and a rotation axis of the first joint member 43 may be spaced apart from each other, and a rotation axis of the second actuator 30 and a rotation axis of the second joint member 33 may also be spaced apart from each other. However, embodiments are not limited thereto, and the actuators 30 and 40 and the joint members 33 and 43 may share a rotation axis. In an embodiment, the actuators 30 and 40 may be spaced apart from the joint members 33 and 43, respectively. In this case, the driving modules 35 and 45 may further include a power transmission module (not shown) configured to transmit power from the actuators 30 and 40 to the joint members 33 and 43. The power transmission module may be a rotary body, such as a gear, or a longitudinal member, such as a wire, a cable, a string, a spring, a belt, or a chain. However, the scope of embodiments is not limited by the positional relationship between the actuator 30 or 40 and the joint member 33 or 43 and the power transmission structure described above.

In an embodiment, the leg support frame 50 or 55 may support a leg (e.g., a thigh) of the user when the wearable device 100 is worn on the leg of the user. For example, the leg support frame 50 or 55 may transmit power generated by the driving module 35 or 45 to the thigh of the user, and the power may act as an external force to be applied to a leg motion of the user. One end of the leg support frame 50 or 55 may be connected to the joint member 33 or 43 and may rotate and, as the other end of the leg support frame 50 or 55 is connected to a cover 11 or 21 of the thigh fastener 1 or 2, the leg support frame 50 or 55 may transmit the power generated by the driving module 35 or 45 to the thigh of the user while supporting the thigh of the user. For example, the leg support frame 50 or 55 may push or pull the user's thigh. The leg support frame 50 or 55 may extend in a longitudinal direction of the user's thigh. The leg support frame 50 or 55 may be bent to surround at least a portion of the circumference of the user's thigh. For example, an upper portion of the leg support frame 50 or 55 may cover a portion oriented in a lateral direction (e.g., the +y direction or the -y direction) of the user's body, and a lower portion of the leg support frame 50 or 55 may cover a portion oriented in a front direction (e.g., the +x direction) of the user's body. The leg support frames 50 and 55 may include the first leg support frame 55 configured to support the right leg of the user and the second leg support frame 50 configured to support the left leg of the user.

The thigh fastener 1 or 2 may be connected to the leg support frame 50 or 55 and may fasten the leg support frame 50 or 55 to the thigh. The thigh fasteners 1 and 2 may include the first thigh fastener 2 configured to fasten the first leg support frame 55 to the right thigh of the user and the second thigh fastener 1 configured to fasten the second leg support frame 50 to the left thigh of the user. The first thigh fastener 2 may include a first cover 21, a first fastening frame 22, and a first strap 23, and the second thigh fastener 1 may include a second cover 11, a second fastening frame 12, and a second strap 13.

In an embodiment, the cover 11 or 21 may apply torque generated by the driving module 35 or 45 to the user's thigh. For example, the cover 11 or 21 may be disposed on one side of the user's thigh and may push or pull the user's thigh. For example, the cover 11 or 21 may be disposed on a front surface of the user's thigh. The cover 11 or 21 may be disposed in a circumferential direction of the user's thigh. The cover 11 or 21 may extend to both sides from the other end of the leg support frame 50 or 55 and may include a curved surface corresponding to the thigh of the user. One end of the cover 11 or 21 may be connected to the fastening frame 12 or 22 and the other end of the cover 11 or 21 may be connected to the strap 13 or 23.

In an embodiment, one end of the fastening frame 12 or 22 may be connected to one side of the cover 11 or 21 and the other end may be connected to the strap 13 or 23. For example, the fastening frame 12 or 22 may be disposed to surround at least a portion of the circumference of the user's thigh and may prevent the user's thigh from being separated from the leg support frame 50 or 55. The first fastening frame 22 may have a fastening structure that connects the first cover 21 to the first strap 23 and the second fastening frame 12 may have a fastening structure that connects the second cover 11 to the second strap 13.

The strap 13 or 23 may surround a remaining portion that is not covered by the cover 11 or 21 and the fastening frame 12 or 22 in the circumference of the user's thigh and may include an elastic material (e.g., a band).

In an embodiment, the wearable device 100 may support a proximal part and a distal part of the user and may assist a relative motion between the proximal part and the distal part. Among the components of the wearable device 100, components worn on the proximal part of the user may be referred to as a "proximal wearing unit" and components worn on the distal part of the user may be referred to as a "distal wearing unit". For example, among the components of the wearable device 100, the housing 80, the waist support frames 20 and 25, the pair of belts 60, and the auxiliary belt 70 may correspond to a proximal wearing unit, and the thigh fasteners 1 and 2 may correspond to a distal wearing unit. For example, the proximal wearing unit may be worn on a waist or a pelvis of the user, and the distal wearing unit may be worn on a thigh or a calf of the user. Positions in which the proximal wearing unit and the distal wearing unit are worn are not limited thereto. For example, the proximal wearing unit may be worn on a torso or a shoulder of the user, and the distal wearing unit may be worn on an upper arm or a lower arm of the user.

FIGS. 5A and 5B are diagrams illustrating a configuration of a control system of a wearable device according to an embodiment.

Referring to FIG. 5A, a wearable device (e.g., the wearable device 100) may be controlled by a control system 500. The control system 500 may include the control module 510, the communication module 516, the sensor module 520, a driving module 530, an input module 540, and the sound output module 550. The driving module 530 may include a motor 534 configured to generate power (e.g., torque) and a motor driver circuit 532 configured to drive the motor 534. The sensor module 520 may include at least one sensor. The sensor module 520 may obtain sensor data including motion information of a user or motion information of the wearable device. The sensor module 520 may transmit the obtained sensor data to the control module 510.

Although FIG. 5A illustrates the driving module 530 including one motor driver circuit 532 and one motor 534, the example of FIG. 5A is merely an example. Referring to FIG. 5B, in a control system 500-1 as shown in the embodiment of FIG. 5B, a plurality (e.g., two or more) of motor driver circuits 532 and 532-1 and motors 534 and 534-1 may be provided. The driving module 530 including the motor driver circuit 532 and the motor 534 may correspond to the first driving module 45 of FIG. 3 and the driving module 530-1 including the motor driver circuit 532-1 and the motor 534-1 may correspond to the second driving module 35 of FIG. 3. The following descriptions of the motor driver circuit 532 and the motor 534 may also be respectively applicable to the motor driver circuit 532-1 and the motor 534-1 shown in FIG. 5B.

The sensor module 520 of FIG. 5A may include the IMU 522 and an angle sensor (e.g., the first angle sensor 520 and the second angle sensor 520-1) as shown in FIG. 5B. The IMU 522 may measure a motion value of the upper body of the user. For example, the IMU 522 may sense X-axis, Y-axis, and Z-axis accelerations and X-axis, Y-axis, and Z-axis angular velocities according to a motion of the user.

The angle sensor may measure a hip joint angle value according to a leg motion of the user. Sensor data that may be measured by the angle sensor may include, for example, a hip joint angle value of a right leg, a hip joint angle value of a left leg, and information on a motion direction of a leg. The first angle sensor 520 of FIG. 5B may obtain a hip joint angle value of the right leg of the user and the second angle sensor 520-1 may obtain a hip joint angle value of the left leg of the user. For example, the first angle sensor 520 and the second angle sensor 520-1 may each include, for example, an encoder and/or a hall sensor. In addition, the angle sensor may obtain a motion value of a leg support frame of the wearable device. For example, the first angle sensor 520 may obtain a motion value of the first leg support frame 55 and the second angle sensor 520-1 may obtain a motion value of the second leg support frame 50.

Referring back to FIG. 5A, in an embodiment, the sensor module 520 may obtain sensor data for estimating a walking index of the user. For example, the sensor data may include walking motion information on a motion of the user when the user walks while wearing the wearable device. The wearable device may transmit the sensor data including the walking motion information of the user to an electronic device (e.g., the electronic device 210 of FIG. 2) linked with the wearable device under the control of a processor 512. The electronic device may estimate the walking index of the user based on the walking motion information.

In an embodiment, the sensor module 520 may further include a position sensor configured to obtain a position value of the wearable device, a proximity sensor configured to sense the proximity of an object, a biosignal sensor configured to detect a biosignal of a user, and a temperature sensor configured to measure an ambient temperature.

The input module 540 may receive a command or data to be used by another component (e.g., a processor 512) of the wearable device from the outside (e.g., the user) of the wearable device. The input module 540 may include, for example, a key (e.g., a button) or a touch screen.

The sound output module 550 may output a sound signal to the outside of the wearable device. The sound output module 550 may include a guide sound signal (e.g., a driving start sound or an operation error notification sound) and a speaker for playing music content or guiding voice. In an embodiment, the sound output module 550 may output the guide for measuring the exercise ability of the user and/or the exercise ability information of the user to the outside of the wearable device under the control of the processor 512.

In an embodiment, the control system 500 may include a battery (not shown) to supply power to each component of the wearable device. The wearable device may convert the power of the battery into power suitable for an operating voltage of each component of the wearable device and supply the converted power to each component.

The driving module 530 may generate an external force to be applied to the user's legs by control of the control module 510. The driving module 530 may be in a position corresponding to a position of a hip joint of the user and may generate torque to be applied to the user's legs based on a control signal generated by the control module 510. The control module 510 may transmit the control signal to the motor driver circuit 532, and the motor driver circuit 532 may control an operation of the motor 534 by generating a current signal (or a voltage signal) corresponding to the control signal and supplying the current signal (or the voltage signal) to the motor 534. The current signal may not be supplied to the motor 534 according to the control signal. When the current signal is supplied to the motor 534, and the motor is driven, the motor 534 may generate a force to assist a leg motion of the user or torque to impede the leg motion of the user.

In an embodiment, the driving module 530 may change the magnitude of a resistance force applied to the user, under the control of the processor 512. For example, when a preset time has arrived or an exercise session has changed in an exercise program performed by the user, the processor 512 may increase the magnitude of the resistance force. In an embodiment, the wearable device may receive the control signal from the electronic device linked with the wearable device via the communication module 516 and thereby, may control the driving module to change the magnitude of the resistance force.

The control module 510 may control an overall operation of the wearable device, and may generate a control signal to control each component (e.g., the driving module 530). The control module 510 may include a processor 512 and a memory 514.

The processor 512 may execute, for example, software to control at least one other component (e.g., a hardware or software component) of the wearable device connected to the processor 512 and may perform various types of data processing or operations. According to an embodiment, as at least a part of data processing or computation, the processor 512 may store instructions or data received from another component (e.g., the communication module 516) in the memory 514, may process the instructions or the data stored in the memory 514, and may store result data in the memory 514. According to an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of, or in conjunction with, the main processor. The auxiliary processor may be implemented separately from the main processor or as a part of the main processor.

The memory 514 may store a variety of data used by at least one component (e.g., the processor 512) of the control module 510. The variety of data may include, for example, software, sensor data, input data, or output data for instructions related thereto. The memory 514 may include a volatile memory or a non-volatile memory (e.g., a random-access memory (RAM), a dynamic RAM (DRAM), or a static RAM (SRAM)).

The communication module 516 may support establishing a direct (e.g., wired) communication channel or a wireless communication channel between the control module 510 and another component of the wearable device or an external electronic device (e.g., the electronic device 210 or the other wearable device 220 of FIG. 2), and performing communication via the established communication channel. For example, the communication module 516 may transmit the sensor data obtained by the sensor module 520 to an external electronic device (e.g., the electronic device 210 of FIG. 2) and receive a control signal from the electronic device. According to an embodiment, the communication module 516 may include at least one CP, which operates independently of the processor 512 and is configured to support direct (e.g., wired) communication or wireless communication.

In an embodiment, the communication module 516 may control a control signal of the electronic device for the exercise ability measurement in response to a user input by the user. The communication module 516 may cause the electronic device to assess the exercise ability information of the user based on the sensor data by transmitting the sensor data including the motion information of the user obtained by the sensor module 520 to the electronic device, under the control of the processor 512.

According to an embodiment, the communication module 516 may include a wireless communication module (e.g., a cellular communication module, a short-range wireless communication module, or a global navigation satellite system (GNSS) communication module), and/or a wired communication module. A corresponding one of these communication modules may communicate with another component of the wearable device and/or an external electronic device via a short-range communication network, such as Bluetooth^{™}, wireless-fidelity (Wi-Fi), an **ANT** or infrared data association (IrDA), or a long-range communication network, such as a legacy cellular network, a 5G network, a next-generation communication network, the Internet, or a computer network (e.g., a local area network (LAN) or a wide region network (WAN).

A wearable device in an embodiment may include the driving module 530 configured to generate torque applied to the body of a user, a support frame configured to support the body of the user and transmit torque generated by the driving module 530 to the body of the user when the wearable device is worn on the body of the user, the sensor module 520 configured to obtain sensor data including motion information of the wearable device, and the control module 510 configured to control the driving module 530 based on the sensor data. The support frame may include, for example, a first leg support frame (e.g., the first leg support frame 55) configured to support the right leg of the user and a second leg support frame (e.g., the second leg support frame 50) configured to support the left leg of the user. The support frame may further include a waist support frame (e.g., the waist support frames 20 and 25) configured to support the waist of the user. The sensor module 520 may include, for example, an IMU (e.g., the IMU 522) configured to obtain a motion value of the waist support frame of the wearable device and an angle sensor (e.g., the first angle sensor 520 and the second angle sensor 520-1) configured to obtain a motion value of the leg support frame of the wearable device. The motion value of the waist support frame may correspond to a motion value of the upper body of the user and the motion value of the leg support frame may correspond to a motion value of the leg of the user.

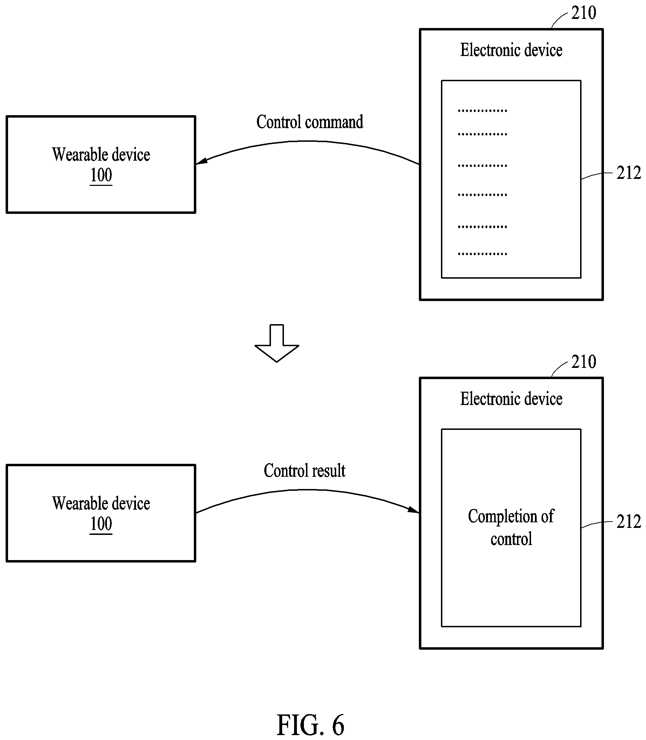

FIG. 6 is a diagram illustrating an interaction between a wearable device and an electronic device according to an embodiment.

Referring to FIG. 6, the wearable device 100 may communicate with the electronic device 210. For example, the electronic device 210 may be a dedicated controller for the wearable device 100 or a user terminal of a user who uses the wearable device 100. According to an embodiment, the wearable device 100 and the electronic device 210 may be connected using short-range wireless communication (e.g., Bluetooth communication).

In an embodiment, the electronic device 210 may check a state of the wearable device 100 or may execute an application to control the wearable device 100 and in response to the execution of the application, a UI screen for controlling an operation of the wearable device 100 or measuring the exercise ability of the user may be displayed on a display 212 of the electronic device 210. In an embodiment, the user may input a command (e.g., a command to execute a walking assistance mode, an exercise assistance mode, or an exercise ability measurement mode) to control an operation of the wearable device 100 or may change settings of the wearable device 100 through a UI screen on the display 212 of the electronic device 210.

The electronic device 210 may generate a control command (or control signal) corresponding to an operation control command or a setting change command input by the user and transmit the generated control command to the wearable device 100. The wearable device 100 may operate according to the received control command and may transmit a control result and/or measured data (e.g., sensor data) to the electronic device 210. The electronic device 210 may provide the user with result information (e.g., walking ability information, exercise posture information, exercise ability information, and physical ability information) derived by analyzing the data of the wearable device 100 and/or the control result through the display 212.

For example, the electronic device 210 may transmit a control command to obtain sensor data to the wearable device in response to a user input for measuring the exercise ability of the user, and the wearable device may transmit the sensor data including the motion information of the user to the electronic device 210 in response to the control command from the electronic device.

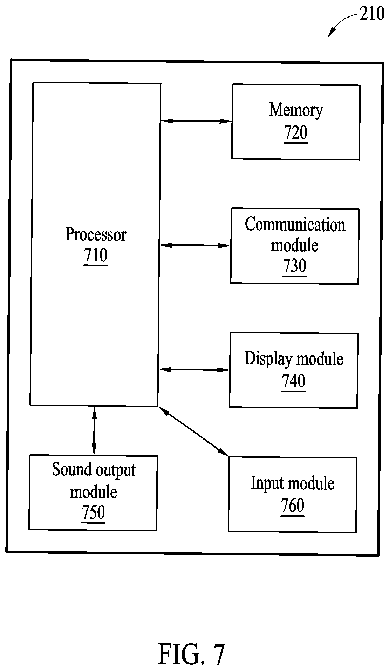

FIG. 7 is a diagram illustrating a configuration of an electronic device according to an embodiment.

Referring to FIG. 7, the electronic device 210 may include a processor 710, a memory 720, a communication module 730, a display module 740, a sound output module 750, and an input module 760. In some embodiments, one or more components (e.g., an input module, a sensor module, and a battery) may be added to the electronic device 210. The electronic device 210 may be linked with a wearable device (e.g., the wearable device 100 of FIG. 1) worn on the body of a user.

The processor 710 may control at least one component (e.g., a hardware or software component) of the electronic device 210 connected to the processor 710 and may perform various types of data processing or operations. According to an embodiment, as at least a part of data processing or operations, the processor 710 may store instructions or data received from another component (e.g., the communication module 730) in the memory 720, process the instructions or the data stored in the memory 720, and store result data in the memory 720.

According to an embodiment, the processor 710 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a CP) that is operable independently of or in conjunction with the main processor.

The memory 720 may store a variety of data used by at least one component (e.g., the processor 710 or the communication module 730) of the electronic device 210. The data may include, for example, a program (e.g., an application), and input data or output data for a command related thereto. The memory 720 may include at least one instruction executable by the processor 710. The memory 720 may include a volatile memory or a non-volatile memory.

The communication module 730 may support establishing a direct (e.g., wired) communication channel or a wireless communication channel between the electronic device 210 and another electronic device (e.g., the wearable device 100, the other wearable device 220, or the server 230) and performing communication via the established communication channel. The communication module 730 may include one or more CPs that are operable independently of the processor 710 (e.g., an AP) and that support direct (e.g., wired) communication or wireless communication.

According to an embodiment, a communication module 730 may include a wireless communication module (e.g., a Bluetooth communication module, a cellular communication module, a short-range wireless communication module, or a GNSS communication module) that performs wireless communication, or a wired communication module (e.g., a LAN communication module, or a power line communication (PLC) module). A corresponding one of these communication modules may communicate with another electronic device via a first communication network (e.g., a short-range communication network, such as Bluetooth^{™}, wireless-fidelity (Wi-Fi) direct, or infrared data association (IrDA)) or a second communication network (e.g., a long-range communication network, such as a legacy cellular network, a 5G network, a next-generation communication network, the Internet, or a computer network (e.g., a LAN or a wide area network (WAN)). For example, the communication module 730 may transmit a control instruction to the wearable device 100 and may receive, from the wearable device, at least one of sensor data including motion information of the user who wears the wearable device 100, state data of the wearable device 100, or control result data corresponding to the control instruction.

In an embodiment, in response to a user input for measuring the exercise ability information, the electronic device 210 may transmit a control signal that enables an exercise ability assessment mode to the wearable device 100 via the communication module 730. For example, the exercise ability information may include information on at least one of the flexibility, symmetry of flexibility, physical strength, symmetry of strength, core stability, or cardiorespiratory endurance of the user.

The wearable device 100 may obtain sensor data from the sensor module in response to the control signal of the electronic device 210. The sensor data may include a body motion value of the user obtained by the IMU included in the wearable device 100 or motion information on a motion of the user when the user performs an exercise or walks while wearing the wearable device 100. The wearable device 100 may transmit the obtained sensor data to the electronic device 210.

The processor 710 may assess an exercise posture of the user for a reference exercise posture defined by at least one exercise program provided to the user based on the sensor data obtained from the wearable device 100 and may determine the exercise ability information of the user based on a result of the assessment. For example, the at least one exercise program may be an exercise program for repeating a standing knee-to-chest motion or a knee-up motion multiple times. In this case, the standing knee-to-chest motion or the knee-up motion may be the reference exercise posture defined by the exercise program.

In an embodiment, the reference exercise posture may include a plurality of posture items. The processor 710 may determine an assessment result of each of the plurality of posture items based on an assessment criterion defined for each of the plurality of posture items based on the sensor data obtained from the wearable device 100. For example, the plurality of posture items may include at least one of the number of motions, the motion velocity, a hip joint angle, or the body stability during the exercise. The processor 710 may assess the body stability of the user based on a body motion value of the user included in the sensor data. The method of the electronic device 210 to assess each of the plurality of posture items is further described with reference to FIGS. 10 and 11.

In an embodiment, the processor 710 may estimate a walking index of the user based on walking motion information of the user included in the sensor data obtained from the wearable device 100. For example, the processor 710 may estimate a walking index including at least one of walking symmetry of the user, a stride length, walking speed, a short physical performance battery (SPPB) test score, or a timed up and go (TUG) test score.

The display module 740 may visually provide information to the outside (e.g., a user) of the electronic device 210. The display module 740 may include, for example, a display, a hologram device, or a projector and control circuitry to control a corresponding one of the display, the hologram device, and the projector. For example, the display module 740 may include a touch sensor adapted to sense a touch, or a pressure sensor adapted to measure the intensity of a force incurred by the touch. In an embodiment, the electronic device 210 may output a UI screen to guide the exercise ability measurement through the display module 740 and after the exercise ability measurement is completed, the electronic device 210 may output exercise ability measurement information.

The sound output module 750 may output a sound signal to the outside of the electronic device 210. The sound output module 750 may include a speaker configured to play back a guiding sound signal (e.g., an operation start sound or an operation error alarm), music content, or a guiding voice based on the state of the wearable device 100. For example, when it is determined that the wearable device 100 is not properly worn on the body of the user, the sound output module 750 may output a guiding voice to notify the user of the abnormal wearing of the wearable device 100 or guide the user to wear the wearable device 100 normally. In an embodiment, the electronic device 210 may output a guide for notifying the start, progress, or end of the exercise ability measurement as a sound signal through the sound output module 750.

The input module 760 may receive a command or data to be used by a component (e.g., the processor 710) of the electronic device 210, from the outside (e.g., the user) of the electronic device 210. The input module 760 may include, for example, a key (e.g., a button) or a touch screen. For example, the user may input a user input for measuring the exercise ability information through the input module 760.

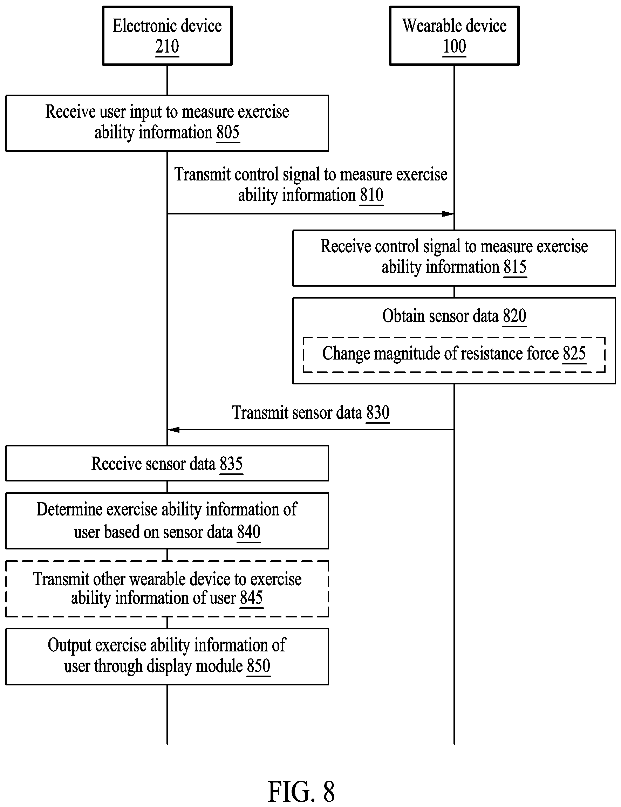

FIG. 8 is a diagram illustrating an operation method of an electronic device for determining exercise ability information of a user according to an embodiment. In an embodiment, at least one of the operations of FIG. 8 may be simultaneously or parallelly performed with one another, and the order of the operations may be changed. In addition, at least one of the operations may be omitted, or another operation may be additionally performed.

In an embodiment, the electronic device 210 may be linked with the wearable device 100 worn on the body of a user. The electronic device 210 may transmit a control command or a control signal to the wearable device 100 via a communication module, and the wearable device 100 may transmit a control result or measured data (e.g., sensor data) to the electronic device 210 via the communication module.

Referring to FIG. 8, in operation 805, the electronic device 210 may receive a user input to measure exercise ability information of a user. The electronic device 210 may include an input module, such as a key (e.g., a button) or a touch screen, and the user may input an exercise ability information measurement test that the user desires to perform on an application executed by the electronic device 210 through the input module.

In operation 810, in response to the user input, the electronic device 210 may transmit a control signal to measure the exercise ability information to the wearable device 100. In operation 815, the wearable device 100 may receive the control signal to measure the exercise ability information from the electronic device 210. In an embodiment, the wearable device 100 may receive, from the electronic device 210, information on a type of exercise ability measurement test to be performed and an assessment factor set by the user for the exercise ability measurement test.

In operation 820, in response to the transmission of the control signal, the wearable device 100 may obtain sensor data from a sensor module. In an embodiment, the sensor data may include exercise information on a motion performed by the user according to at least one exercise program provided to the user when the wearable device 100 operates in the exercise ability assessment mode. For example, the sensor data may include a body motion value of the user obtained by an IMU included in the wearable device 100 and may include walking motion information on a motion of the user when the user walks while wearing the wearable device 100.

In operation 825, the wearable device 100 may change a magnitude of a resistance force applied to the user based on the obtained sensor data. For example, when a defined condition is satisfied while the user moves according to the exercise ability assessment mode, the processor included in the wearable device 100 may control the driving module to change the magnitude of the resistance force applied to the user. In an embodiment, the wearable device 100 may control the driving module to change the magnitude of the resistance force by receiving a control signal in response to the user input by the user from the electronic device 210 via the communication module. However, operation 825 may be omitted depending on the embodiment.

In operation 830, the wearable device 100 may transmit the obtained sensor data to the electronic device 210 to cause the electronic device 210 to assess the exercise ability of the user based on the sensor data and determine the exercise ability information of the user.

In operation 835, the electronic device 210 may obtain the sensor data including the motion information of the user to assess the exercise ability of the user from the wearable device 100.

In operation 840, the electronic device 210 may determine the exercise ability information of the user based on the sensor data. For example, the electronic device 210 may determine the exercise ability information of the user based on at least one of the exercise information on a motion performed by the user according to at least one program provided to the user, a body motion value of the user obtained by the IMU, or walking motion information on a motion when the user walks.

In an embodiment, the electronic device 210 may assess an exercise posture of the user for a reference exercise posture defined by at least one program based on the sensor data. The electronic device 210 may determine the exercise ability information based on a result of the assessment. The reference exercise posture may include a plurality of posture items. For example, the plurality of posture items may include at least one of the number of motions, the motion velocity, a hip joint angle, or the body stability during the exercise.

In an embodiment, the electronic device 210 may determine an assessment result of each of the plurality of posture items based on assessment criteria defined for each of the plurality of posture items based on the sensor data obtained from the wearable device 100. For example, when the user performs at least one exercise program, the wearable device 100 may provide the electronic device 210 with motion information on the motion of the user obtained by the sensor module, and the electronic device 210 may calculate the number of motions, the motion velocity, a hip joint angle, or the body stability during the motion while the user performs the exercise program based on the sensor data. The electronic device 210 may assess each of the plurality of posture items by comparing the calculated number of motions, the motion velocity, the hip joint angle, and the body stability during the motion with assessment reference values defined for the plurality of posture items, respectively.

In an embodiment, the electronic device 210 may estimate a walking index of the user based on walking motion information included in the sensor data. The walking motion information on the motion during the walking of the user may include information on acceleration and/or angular velocity on the motion of the user obtained by the IMU included in the wearable device 100 or information on the hip joint angle of the user obtained by an angle sensor included in the wearable device 100. Based on the information on the walking motion of the user, the electronic device 210 may estimate the walking index including at least one of the walking symmetry of the user, walking variability, stride length, walking speed, walking health age, an SPPB test score, and a TUG test score.

In operation 845, the electronic device 210 may transmit walking ability information of the user to another wearable device. The other wearable device may include, for example, wireless earphones (e.g., the wireless earphones 222 of FIG. 2), a smartwatch (e.g., the smartwatch 224 of FIG. 2), or smart glasses (e.g., the smart glasses 226 of FIG. 2), but the example is not limited thereto. However, operation 845 may be omitted depending on the embodiment.

In operation 850, the electronic device 210 may output the exercise ability information of the user through the display module. In an embodiment, the display module may output a UI screen to guide the measurement of the exercise ability of the user and may output the exercise ability information of the user, under the control of the processor of the electronic device 210.

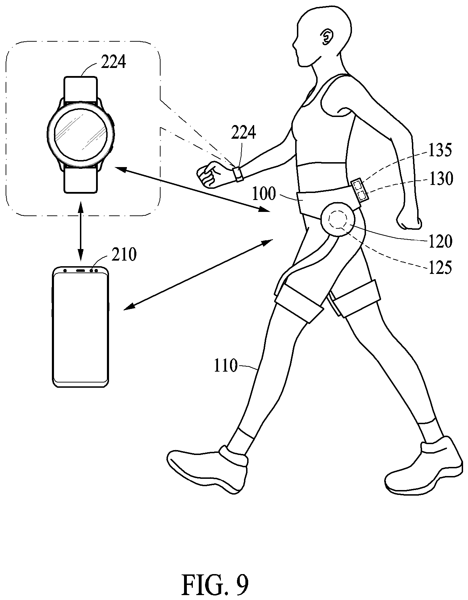

FIG. 9 is a diagram illustrating an interaction among an electronic device, a wearable device, and another wearable device according to an embodiment.

Referring to FIG. 9, the user 110 may wear the wearable device 100 and the smartwatch 224. Depending on the embodiment, the smartwatch 224 shown in FIG. 10 may be replaced with another wearable device (e.g., smart glasses, wireless earphones, etc.) and the embodiment is not limited thereto. For example, the wearable device 100 worn by the user may be the wearable device 100 described with reference to FIG. 1.

In an embodiment, the wearable device 100, the smartwatch 224, and the electronic device 210 may each include a communication module, and the communication module may each include a wireless communication module and/or a wired communication module. For example, each of the communication modules may communicate with each other via a short-range communication network, such as Bluetooth, Wi-Fi, an ANT, or IrDA, or a long-range communication network, such as a legacy cellular network, a 5G network, a next-generation communication network, the Internet, or a computer network (e.g., a LAN or a WAN). In other words, the electronic device 210, the wearable device 100, and the smartwatch 224 may be linked to each other.

The user 110 may input a user input to measure the exercise ability of the user through the wearable device 100 or the smartwatch 224. In an embodiment, the user 210 may command the wearable device 100 to execute the exercise ability measurement mode by inputting a user input to the electronic device 210. The electronic device 210 may output a guide to notify of the start of the exercise ability measurement through a display module or a sound output module in response to the user input and may transmit a control signal to enable the exercise ability assessment mode to the wearable device 100. In addition, the electronic device 210 may output a guide to notify of the exercise ability measurement to the display module of the smartwatch 224 by transmitting the control signal to the smartwatch 224.

In an embodiment, the smartwatch 224 may include a sensor module configured to sense a heartbeat of the user. The smartwatch 224 may obtain heart rate data of the user 110 by sensing a heart rate of the user 110 in real-time. The smartwatch 224 may transmit the obtained heart rate data of the user 110 to the electronic device 210 through the communication module. The electronic device 210 may obtain the heart rate data of the user 110 from the smartwatch 224 and may provide a health state of the user 110 to the user 110 based on the heart rate data.

For example, the user 110 may set a target heart rate (THR) to achieve a maximum exercise effect. A maximum heart rate (HRmax) and a resting heart rate (Resting HR) of the user 110 may be estimated through a biometric sensor provided in the smartwatch 224. The smartwatch 224 or the electronic device 210 may calculate a heart rate reserve (HRR) based on the maximum heart rate and the resting heart rate and may calculate the THR based on the HRR.

When the user 110 performs at least one exercise program to measure the exercise ability, the exercise ability may be more accurately assessed by measuring the heart rate of the user 110 by using the smartwatch 224 in real-time or performing the exercise program by setting the THR.

In an embodiment, the user 110 may use the smartwatch 224 to measure cardiorespiratory endurance. For example, after the exercise program is terminated, the electronic device 210 may notify the smartwatch 224 and/or the wearable device 100 of the termination of the exercise program through a control signal and the smartwatch 224 may provide the electronic device 210 with heart rate data obtained by sensing a maximum heart rate immediately after the termination of the exercise program and a heart rate one minute after the termination of the exercise program. The electronic device 210 may calculate a recovery heart rate (recovery HR) of the user 110 based on the heart rate data obtained by the smartwatch 224 and may assess the cardiorespiratory endurance of the user 110 based on the recovery heart rate.

In an embodiment, when the user 110 does not wear the other wearable device that is able to measure a heart rate of the user 110 in real-time, for example, the smartwatch 224, the electronic device 210 may estimate the cardiorespiratory endurance of the user 110 based on a rating of perceived exertion (RPE) selected by the user 110 after the exercise.

**[Table 1]**

| **Level of exertion** | **Borg scale** | **Estimated heart rate** |
|---|---|---|
| No exertion at all | 0 | 60 |
| Extremely light | 1 | 70 |
| Very light | 2 | 85 |
| Light | 3 | 100 |
| | 4 | 110 |
| Somewhat hard | 5 | 125 |
| Hard (heavy) | 6 | 140 |
| | 7 | 155 |
| Very hard | 8 | 170 |
| | 9 | 180 |
| Extremely hard | 10 | 195 |
| Maximal exertion | | |

Referring to Table 1, the user 110 may assess a level of exertion subjectively felt by the user 110 immediately after the termination of the exercise program and one minute after the termination of the exercise program. For example, when the user 110 does not feel hard after the termination of the exercise, the user 110 may assess as No exertion at all. When the user feels quite hard, the user 110 may assess as Somewhat hard, and when the user feels maximally hard, the user 110 may assess as Maximal exertion. Based on the level of exertion assessed by the user 110, different Borg scales may be assigned and a heart rate for the exercise of the user 110 may be estimated based on the Borg scale. For example, based on the level of exertion felt by the user 110, one of the Borg scales from 0 to 10 may be assigned and an expected heart rate of the user 110 may be estimated based on the assigned Borg scale. The electronic device 210 may estimate the cardiorespiratory endurance of the user 110 based on the estimated heart rate.

FIG. 10 is a diagram illustrating a process of determining exercise ability information of a user when the user wears a wearable device and performs a standing knee-to-chest exercise, according to an embodiment.

Referring to FIG. 10, the user 110 may wear the wearable device 100 and may perform exercise provided by a first exercise program. The electronic device may estimate the exercise ability of the user 110 based on sensor data obtained during an exercise process of the user 110 by linking with the wearable device 100.

For example, the first exercise program may be an exercise program for repeating a standing knee-to-chest motion multiple times. A reference exercise posture defined by the first exercise program may be a second posture 1020. To perform a standing knee-to-chest motion, the user 110 may alternately pull the left knee and the right knee of the user 110 to their chest and maintain the posture for a predetermined time. However, the exercise type performed in the first exercise program may vary depending on the embodiment.

Before performing the first exercise program, the user 110 may perform a first posture 1010, which is a standing posture. In response to a user input to measure the exercise ability of the user 110, when a guide to start the first exercise program is output through a display module or a sound output module of the electronic device 210, the user 110 may maintain the first posture 1010 and then may perform the standing knee-to-chest motion, which is the second posture 1020.

The user 110 may receive, from the electronic device, a guide to maintain the second posture 1020 for a predetermined time. While the user 110 performs the first exercise program, the wearable device 100 may obtain sensor data including motion information of the user 110 through a sensor module. For example, the sensor module may include the angle sensor 125 and the IMU 135, the angle sensor 125 may measure a hip joint angle value of the user 110, and the IMU 135 may measure a motion value of the upper body of the user 110. The IMU 135 may sense X-axis, Y-axis, and Z-axis accelerations and X-axis, Y-axis, and Z-axis angular velocities according to a motion of the user.

The user 110 may assess the flexibility and/or flexibility symmetry of the user 110 among the exercise ability information by performing the first exercise program. For example, the reference exercise posture defined by the first exercise program may include at least one of the number of motions, the motion velocity, a hip joint angle, or the body stability during the exercise.

The electronic device 210 may count the number of motions by determining that an exercise attempt has been made regardless of whether the user's posture is correct based on the hip joint angle value of the user obtained through an angle sensor (e.g., an encoder and/or a hall sensor) included in the wearable device 100. Referring to a reference number 1030, when the user 110 performs exercise in the first exercise program, the wearable device 100 may measure a hip joint angle value θ of the user 110. For example, when a hip joint angle value corresponding to the right leg of the user 110 or a hip joint angle value corresponding to the left leg of the user 110 exceeds 15 degrees while the user 110 performs the first exercise program, the electronic device may count that the user 110 performs the standing knee-to-chest exercise once.

In an embodiment, the electronic device 210 may assess the motion velocity for each exercise to determine whether the user performs the exercise at an appropriate speed. For example, one of (1) fast speed, (2) intermediate speed, and (3) slow speed may be set for each exercise and the user may perform the exercise by selecting one of the three speeds. An execution time for one motion, which is a standard, may be set to each speed. When the user selects the speed and performs the exercise, the assessment of the motion velocity of the user may be better as an execution time performed by the user is close to the execution time for one motion, which is the standard for the speed that the user selects.

In an embodiment, the electronic device 210 may assess the hip joint angle of the user to determine whether the user performs the exercise in an appropriate motion range or an appropriate stride length. Referring to the reference number 1030, when the user 110 performs the standing knee-to-chest motion, the wearable device 100 may measure the hip joint angle value of the user 110.

In an embodiment, a hip joint angle reference value, which is an assessment criterion of the hip joint angle of the user in the standing knee-to-chest motion, may be defined. When the user 110 performs the standing knee-to-chest motion once, the electronic device 210 may determine a maximum value among hip joint angle values of the user 110 measured by the angle sensor 125 to be a hip joint angle value during the standing knee-to-chest motion of the user 110. The electronic device may assess the hip joint angle of the user 110 by comparing the hip joint angle value during the motion of the user 110 with the hip joint angle reference value and the assessment of the hip joint angle of the user may be better as the hip joint angle value during the motion of the user approaches the hip joint angle reference value.

In an embodiment, the body stability during the exercise may include front and rear stability, lateral stability, and rotational stability. While the user 110 performs the first exercise program, the IMU 135 of the wearable device 100 may measure X-axis, Y-axis, and Z-axis accelerations and angular velocities according to the motion of the user. In response to a user input to measure the exercise ability of the user 110, the wearable device 100 may transmit the sensor data including the motion information of the user 110 to the electronic device. The electronic device may assess the body stability during the exercise of the user 110 based on the information on the acceleration and angular velocity according to the motion of the user 110 included in the sensor data.

Referring to a reference number 1040, the electronic device may assess the front and rear stability of the user 110 when the user 110 performs exercise in the first exercise program. In an embodiment, in the standing knee-to-chest motion, a front and rear inclination reference value, which is an assessment criterion of the front and rear stability of the user 110, may be defined. The IMU 135 may measure an inclination value in which the pelvis of the user 110 tilts forward or backward during the standing knee-to-chest motion of the user 110. When the user 110 performs the standing knee-to-chest motion once, the electronic device may determine a maximum value among the front and rear inclination values measured by the IMU 135 to be a front and rear stability value when the user 110 performs the standing knee-to-chest motion once. The electronic device may assess the front and rear stability of the user 110 by comparing the front and rear stability value during the motion of the user 110 with a front and rear inclination reference value. As the front and rear stability value during the motion of the user approaches the front and rear reference value, the assessment of the front and rear stability of the user may be better.

Referring to a reference number 1050, the electronic device may assess the lateral stability of the user 110 when the user 110 performs exercise in the first exercise program. In an embodiment, in the standing knee-to-chest motion, a lateral inclination reference value, which is an assessment criterion of the lateral stability of the user 110, may be defined. The IMU 135 may measure an inclination value in which the pelvis of the user 110 tilts to the right or left during the standing knee-to-chest motion of the user 110. When the user 110 performs the standing knee-to-chest motion once, the electronic device may determine a maximum value among lateral inclination values measured by the IMU 135 to be a lateral stability value when the user 110 performs the standing knee-to-chest motion once. The electronic device may assess the lateral stability of the user 110 by comparing the lateral stability value during the motion of the user 110 with a lateral inclination reference value. As the lateral stability value during the motion of the user approaches the lateral reference value, the assessment of the lateral stability of the user may be better.

Referring to a reference number 1060, the electronic device may assess the rotational stability of the user 110 when the user 110 performs exercise in the first exercise program. In an embodiment, in the standing knee-to-chest motion, a rotational inclination reference value, which is an assessment criterion of the rotational stability of the user 110, may be defined. When the user 110 performs the standing knee-to-chest motion, the IMU 135 may measure a rotation value of the pelvis of the user 110. When the user 110 performs the standing knee-to-chest motion once, the electronic device may determine a maximum value among rotation values measured by the IMU 135 to be a rotation value when the user 110 performs the standing knee-to-chest motion once. The electronic device may assess the rotational stability of the user 110 by comparing the rotation value during the motion of the user 110 with a rotation reference value. As the front and rear stability value during the motion of the user approaches the front and rear reference value, the assessment of the front and rear stability of the user may be better.

**[Table 2]**

| | Motion velocity (Normal velocity) | Hip joint angle | Front and rear stability | Lateral stability | Rotational stability |
|---|---|---|---|---|---|
| Reference value | 6 seconds | 120° | Pelvis inclination angle + 10° | Parallel | Parallel |
| Top | Reference value ± 1 second | Reference value ± 10° | Reference value ± 3° | Reference value ± 10° | Reference value ± 10° |
| Intermediate | Reference value ± 2 seconds | Reference value ± 20° | Reference value ± 5° | Reference value ± 20° | Reference value ± 20° |
| Bottom | Other ranges | Other ranges | Other ranges | Other ranges | Other ranges |

Referring to Table 2, a reference value to be an assessment criterion may be defined for each of a plurality of posture items while the user 110 performs the standing knee-to-chest motion in an exercise program. The plurality of posture items may include, for example, the motion velocity, a hip joint angle, the front and rear stability, the lateral stability, and the rotational stability, but the example is not limited thereto. In an embodiment, when the user 110 sets the motion velocity to the normal velocity before performing the exercise, a reference value of the normal velocity may be defined as 6 seconds, and when the time taken for the user 110 to perform the motion once approaches 6 seconds, the assessment of the motion velocity may approach "top". The assessment reference value for each of the plurality of posture items may vary depending on the settings of the user 110.

For example, the electronic device 210 may assess the flexibility and the flexibility symmetry based on the sensor data obtained by the wearable device 100 while the user 110 performs the standing knee-to-chest motion in the exercise program. For example, while the user 110 performs the standing knee-to-chest motion, after the electronic device 210 calculates maximum values of hip joint angles of the right leg and left leg of the user 110, the electronic device 210 may assess the flexibility based on an average value of the maximum values of hip joint angles in each motion. As the average value of maximum values of hip joint angles increases, the flexibility may be assessed to be good.

In an embodiment, while the user 110 performs the standing knee-to-chest motion, after the electronic device 210 calculates maximum values of hip joint angles of the right leg and left leg of the user 110, the electronic device 210 may assess the flexibility symmetry by calculating a difference between the maximum value of hip joint angle of the right leg and the maximum value of hip joint angle of the left leg.

FIG. 11 is a diagram illustrating a process of determining exercise ability information of a user when the user wears a wearable device and performs a knee-up exercise, according to an embodiment.

Referring to FIG. 11, the user 110 may perform the knee-up exercise while wearing the wearable device 100. The electronic device 210 may be linked with the wearable device 100 and may estimate the exercise ability of the user 110 based on the sensor data obtained during the knee-up exercise of the user. The knee-up exercise may be an exercise for alternately performing a first posture 1110, which is a standing posture with legs, and a second posture 1120, which is a posture with one leg raised. In the knee-up exercise, a reference exercise posture, which is the subject of assessment of the exercise ability, may be the second posture 1120.

In an embodiment, in the beginning, the user 110 may fix their left foot to the floor while raising the right knee of the user 110 as the first posture 1110 to perform motions of the knee-up exercise. Thereafter, after the user 110 raises the right knee to the chest as the second posture 1120, the user 110 may lower the right knee to the floor as the first posture 1110, and then the user 110 may fix the left foot to the floor while raising the right knee. The user 110 may alternately raise the left knee and the right knee. However, the exercise type performed in a second program may vary depending on the embodiments.

The user 110 may perform the first posture 1110, which is a standing posture, before performing the second exercise program. In response to a user input to measure the exercise ability of the user 110, when a guide to start the second exercise program is output from the electronic device 210 through a display module or a sound output module of the electronic device 210, the user 110 may maintain the first posture 1110 and then may perform the knee-up motion, which is the second posture 1120. After performing the second posture 1120, the user 110 may return to the first posture 1110, which is the standing posture, and may perform the second posture 1120 again with a leg that is opposite to the leg performing the second posture 1120 previously. The user 110 may iteratively perform the first posture 1110 and the second posture 1120 by alternating the right leg and the left leg of the user 110.

While the user 110 performs the second exercise program, the wearable device 100 may obtain the sensor data including motion information of the user 110 through the sensor module and may transmit the obtained sensor data to the electronic device 210. For example, the sensor module may include the angle sensor 125 and the IMU 135, the angle sensor 125 may measure a hip joint angle value of the user 110, and the IMU 135 may measure a motion value of the upper body of the user 110. The IMU 135 may sense X-axis, Y-axis, and Z-axis accelerations and X-axis, Y-axis, and Z-axis angular velocities according to a motion of the user.

The user 110 may assess at least one of the physical strength, strength symmetry, and core stability of the user 110 among the exercise ability information by performing the second exercise program. For example, the reference exercise posture defined by the second exercise program may include at least one of the number of motions, the motion velocity, a hip joint angle, or the body stability during the exercise.

The electronic device 210 may count the number of motions by determining that an exercise attempt has been made regardless of whether the user's posture is correct based on the hip joint angle value of the user obtained through an angle sensor (e.g., an encoder and/or a hall sensor) included in the wearable device 100. Referring to a reference number 1030, while the user 110 performs the exercise in the second exercise program, the wearable device 100 may measure a hip joint angle value θ of the user 110. The method of assessing the number of motions, the motion velocity, and the hip joint angle of the user 110 in the second exercise program may be construed with reference to the embodiments described with reference to FIG. 10 above.

In an embodiment, the electronic device 210 may assess the body stability during the exercise of the user 110 among the plurality of posture items, and the body stability may include the front and rear stability, the lateral stability, and the rotational stability. Referring to a reference number 1140, when the user 110 performs the exercise in the second exercise program, the front and rear stability of the user 110 may be assessed by comparing a reference value, which is the assessment criteria of the front and rear stability, with a front and rear stability value of the user 110. The method of assessing the front and rear stability of the plurality of posture items may be construed with reference to embodiments described with reference to FIG. 10 above.

Referring to a reference number 1150, when the user 110 performs the exercise in the second exercise program, the lateral stability of the user 110 may be assessed by comparing a reference value, which is the assessment criteria of the lateral stability, with a lateral stability value of the user 110. The method of assessing the lateral stability of the plurality of posture items may be construed with reference to embodiments described with reference to FIG. 10 above.

Referring to a reference number 1160, when the user 110 performs the exercise in the second exercise program, the rotational stability of the user 110 may be assessed by comparing a reference value, which is the assessment criteria of the rotational stability, with a rotational stability value of the user 110. The method of assessing the rotational stability of the plurality of posture items may be construed with reference to embodiments described with reference to FIG. 10 above.

In an embodiment, while the user 110 performs the second exercise program, the wearable device 100 may apply a resistance force to the user 110 to increase the exercise intensity of the user 110. For example, as the magnitude of the resistance force applied to the user 110 by the wearable device 100 increases, the exercise intensity of the user 110 may increase. While the user 110 performs the second exercise program, the wearable device 100 may control the driving module of the wearable device 100 to change the magnitude of the resistance force applied to the user when a preset condition is satisfied.

In an embodiment, when a preset time has arrived or an exercise session has changed, the wearable device 100 may change the magnitude of the resistance force applied to the user 110. For example, as described above, the electronic device 210 may sense the number of knee-up motions while the user 110 performs the second exercise program, and when the user 110 performs the knee-up motion for a predetermined number of times, the electronic device 210 may control the wearable device 100 to increase the magnitude of the resistance force applied to the user 110 by transmitting a control signal to the wearable device 100. For example, after the user 110 starts the second exercise program, when the preset time has arrived, the electronic device 210 may transmit the control signal to the wearable device 100, and thereby, may control the wearable device 100 to increase the magnitude of the resistance force applied to the user 110.

In an embodiment, the wearable device 100 may not receive the control signal of the electronic device 210 and may change the magnitude of the resistance force applied to the user 110 as the processor of the wearable device 100 controls the driving module.

In an embodiment, while the user 110 performs the knee-up motion in the exercise program, the electronic device 210 may assess, for example, the physical strength, strength symmetry, and core stability, based on the sensor data obtained from the wearable device 100. For example, while the user 110 performs the standing knee-to-chest motion, the electronic device 210 may assess the physical strength based on the hip joint angle of the user 110 and front and rear angle values of the pelvis of the user 110. The electronic device 210 may assess the physical symmetry of the user 110 based on a left muscle strength score of the user 110 and a right muscle strength score of the user 110. The electronic device 210 may assess the core stability of the user 110 based on the hip joint angle, front and rear angles of the pelvis, or the left and right angles of the pelvis of the user 110.

**[Table 3]**

| | Motion velocity (Normal velocity) | Hip joint angle | Front and rear stability | Lateral stability | Rotational stability |
|---|---|---|---|---|---|
| Reference value | 2.5 seconds | 110° | Pelvis inclination angle + 5° | Parallel | Parallel |
| Top | Reference value ± 0.5° | Reference value ± 10° | Reference value ± 3° | Reference value ± 10° | Reference value ± 10° |
| Intermediate | Reference value ± 1.0 seconds | Reference value ± 20° | Reference value ± 5° | Reference value ± 20° | Reference value ± 20° |
| Bottom | Other ranges | Other ranges | Other ranges | Other ranges | Other ranges |

Referring to Table 3, a value to be the assessment criteria for each of the plurality of posture items may be defined while the user 110 performs the knee-up exercise motion in the exercise program. The plurality of posture items may include, for example, the motion velocity, a hip joint angle, the front and rear stability, the lateral stability, and the rotational stability, but the example is not limited thereto.

In an embodiment, when the user 110 sets the motion velocity to the normal velocity before performing the exercise, a reference value of the normal velocity may be defined as 2.5 seconds, and when the time taken for the user 110 to perform the knee-up motion once approaches 2.5 seconds, the assessment of the motion velocity may be better. For example, when the time taken for the user 110 to perform the knee-up motion once is within 2.5 seconds, the user 110 may be assessed as "top" for the motion. When the time taken for the user 110 to perform the knee-up motion once exceeds 2.5 seconds and is within 3.0 seconds, the user 110 may be assessed as "intermediate" and, when the time taken for the user 110 to perform the knee-up motion once exceeds 3.0 seconds, the user 110 may be assessed as "bottom". An assessment reference value for each of the plurality of posture items may vary depending on the embodiments.

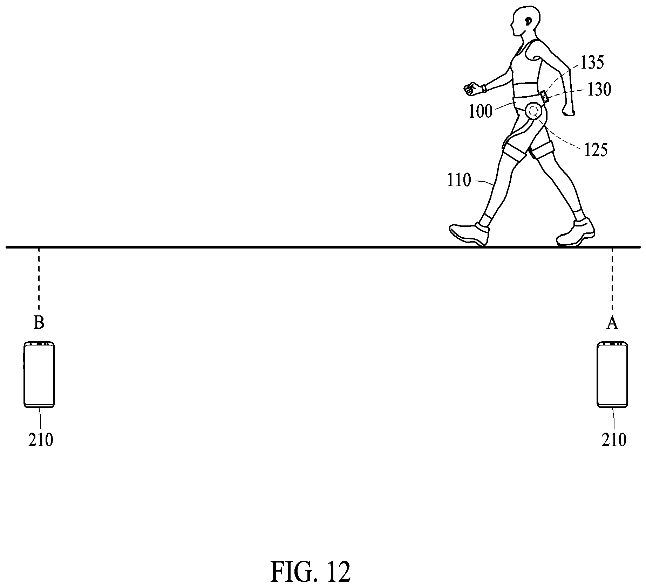

FIG. 12 is a diagram illustrating a process of measuring a walking index of a user using a wearable device according to an embodiment.

Referring to FIG. 12, the user 110 may perform walking from a starting point A of a reference line to an ending point B according to a guide provided by an electronic device (e.g., the electronic device 210 of FIG. 2) linked with the wearable device 100 worn by the user 110. In the walking process, the wearable device 100 may obtain sensor data through one or more sensors (e.g., the angle sensor 125 and the IMU 135) included in the sensor module, and may transmit the obtained sensor data to the electronic device. The electronic device may estimate a walking index of the user 110 based on the sensor data obtained from the wearable device 100. For example, the sensor data may include walking motion information on the motion of the user 110 when the user 110 wears the wearable device 100 and walks.

The walking index may include information about at least one of the pelvis rotation, walking symmetry, walking level using upper body, walking distance, walking interval, stride length, walking speed, walking health age, walking symmetry score, SPPB test score, and TUG test score of the user 110.

The electronic device may calculate front and rear angles or left and right angles of the pelvis of the user 110 based on the sensor data obtained by the IMU of the wearable device 100 and may assess the pelvis rotation based on the calculated front and rear angles or left and right angles of the pelvis of the user 110.

The electronic device may calculate a hip joint stretching ratio and a time ratio during stepping of the right and left feet of the user 110 based on the sensor data obtained by the angle sensor of the wearable device 100. The electronic device may assess the walking symmetry or walking variability based on the calculated hip joint stretching ratio and time ratio during the stepping of the right and left feet of the user 110.

The electronic device may assess the walking level using upper body by calculating the pelvis rotation of the user 110 based on the sensor data obtained by the IMU of the wearable device 100. The electronic device may assess the walking distance by calculating the stepping time of the left and right feet of the user 110 based on the sensor data obtained by the angle sensor of the wearable device 100. The electronic device may assess a walking interval by calculating the stepping time of one of the left and right feet of the user 110 based on the sensor data obtained by the angle sensor of the wearable device 100.

The electronic device may calculate a stride length by calculating a distance from the heel of one foot of the user to the heel of the other foot, based on the sensor data.

The electronic device may assess the walking speed in real-time based on the sensor data obtained by the angle sensor of the wearable device 100. The electronic device may assess the walking health age by calculating the walking speed from the beginning to the end of walking, based on the sensor data obtained by the angle sensor of the wearable device 100. The electronic device may provide a statistical material comprising a walking symmetry score determined based on the sensor data obtained by the wearable device 100.

In an embodiment, the user 110 may perform an SPPB test using the wearable device 100 and the electronic device 210. The electronic device 210 may receive a user input for proceeding with the SPPB test and in response to the user input, the electronic device 210 may request the wearable device 100 to enable an exercise ability assessment mode to perform the SPPB test. In the SPPB test, the user 110 may wear the wearable device 100 and may stand with a specific leg posture, and the wearable device 100 may obtain the sensor data by measuring a degree of the motion of the user 110 during the test time. For example, the sensor data may include walking motion information on the motion of the user 110 when the user 110 wears the wearable device 100 and walks. The wearable device 100 may transmit the sensor data obtained during the test time to the electronic device 210 and the electronic device 210 may estimate an SPPB test score of the user 110 based on the sensor data received from the wearable device 100.

The electronic device 210 may count down the preparation time for proceeding with the SPPB test and the wearable device 100 may initialize the sensor data output from the sensor module during the preparation time. For example, the wearable device 100 may initialize a hip joint angle value output from an angle sensor and an upper body motion value output from an IMU. In an embodiment, the electronic device 210 may provide a guide to a test setting method for an SPPB test to the user and a guide to a motion that the user needs to take while wearing the wearable device 100 before the beginning of the SPPB test.

When the countdown to the preparation time is completed, the electronic device 210 may notify the user of the beginning of the SPPB test and may activate a timer to measure the time.

The electronic device 210 may record a leg motion during a preset time. The electronic device 210 may assess the exercise ability of the user based on the leg motion. The electronic device 210 may estimate a motion level of the leg based on a hip joint angle value and may estimate a motion level of the upper body based on an upper body motion value. When a hip joint angle value measured for one time interval (e.g., 10 seconds) is greater than or equal to a first threshold, the electronic device 210 may determine that the leg of the user moves. When a motion value of the upper body of the user measured for one time interval is greater than or equal to a second threshold, the electronic device 210 may determine that the upper body of the user moves.

In an embodiment, the electronic device 210 may estimate the exercise ability (e.g., balance) of the user based on the posture of the user subjected to the SPPB test, the upper body motion value measured for one time interval, and the determined time in which the leg moves. The electronic device 210 may assign different scores depending on the posture of the user, the upper body motion value, and the time in which the leg moves, and may determine a final score of the SPPB test of the user based on a score of each assessment element. For example, the electronic device 210 may assign a relatively low score when the motion level of the upper body and/or the motion level of the leg is great during the SPPB test. In addition, the electronic device 210 may assign a lower score as the time in which the user moves their leg is fast.

In an embodiment, the electronic device 210 may calculate a score related to the exercise ability assessment of the user based on the assessment criteria to assess the exercise ability of the user through the SPPB test. The motion level of the upper body may be determined based on, for example, an upper body motion value and/or a hip joint angle value.

In an embodiment, the user 110 may perform a TUG test using the wearable device 100 and the electronic device 210. The electronic device 210 may receive a user input to perform the TUG test and in response to the user input, the electronic device 210 may request the wearable device 100 to enable the exercise ability assessment mode to proceed with the TUG test. A chair to sit thereon may be prepared for the TUG test and a predetermined distance from the chair may be displayed. In the TUG test, when the test begins while the user 110 wears the wearable device 100 and sits on the chair, the time taken for the user 110 to get up from the chair, travels the predetermined distance, and return and sit on the chair may be measured. The wearable device 100 may obtain the sensor data by measuring the motion level of the user 110 during the test time. For example, the sensor data may include walking motion information on the motion of the user 110 when the user 110 wears the wearable device 100 and walks. The wearable device 100 may transmit the sensor data obtained during the test time to the electronic device 210 and the electronic device 210 may estimate information on the TUG test based on the sensor data received from the wearable device 100.

The electronic device 210 may count down the preparation time for proceeding with the TUG test and the wearable device 100 may initialize a hip joint angle value output from an angle sensor during the preparation time. For example, the wearable device 110 may set the hip joint angle value output from the angle sensor to "0" during the preparation time. In an embodiment, the electronic device 210 may provide a guide to a preparation item (e.g., a chair and a measuring tape) and a test setting method for a TUG test to the user and a guide to a motion that the user needs to take while wearing the wearable device 100 before the beginning of the TUG test.

When the countdown to the preparation time is completed, the electronic device 210 may notify the user of the beginning of the TUG test and may activate a timer to measure the time. When the user moves their body according to the TUG test method, the wearable device 100 may obtain a hip joint angle value through the sensor module and may transmit the obtained hip joint angle value to the electronic device 210. The electronic device 210 may determine whether the hip joint angle value is greater than a first threshold value. When the hip joint angle value is greater than the first threshold value, the electronic device 210 may recognize that the user gets up from the chair and thereby, may change an operation state value of the user to a first state value.

Thereafter, the electronic device 210 may determine whether the hip joint angle value is less than a second threshold value. In this case, the second threshold value may be different from the first threshold value. For example, the second threshold value may be less than the first threshold value. When the hip joint angle value is less than the second threshold value, the electronic device 210 may recognize that the user sits on the chair again and thereby, may change the operation state value of the user to a second state value.

The electronic device 210 may change the operation state value to a third state value after a predefined time (e.g., 2 seconds) has elapsed. In this case, the first state value, the second state value, and the third state value may be different from each other.

In an embodiment, for the above TUG test, a hip joint angle value corresponding to a right leg, a hip joint angle value corresponding to a left leg, or an average value of the hip joint angle value corresponding to the right leg and the hip joint angle value corresponding to the left leg may be used. In an embodiment, the electronic device 210 may perform the TUG test based on a hip joint angle value obtained by filtering a hip joint angle value.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things, unless the relevant context clearly indicates otherwise. As used herein, "A or B," "at least one of A and B," "at least one of A or B," "A, B or C," "at least one of A, B and C," and "at least one of A, B, or C," may include any one of the items listed together in the corresponding one of the phrases, or all possible combinations thereof. Terms such as "1st" and "2nd," or "first" and "second" may be used to simply distinguish a corresponding component from other components, and do not limit the components in other aspects (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., by wire), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums. Embodiments as set forth herein may be implemented as software including one or more instructions that are stored in a storage medium (e.g., the memory 514) that is readable by a machine. For example, a processor of the machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to embodiments of the disclosure may be included and provided in a computer program product. The computer program product may be traded as a product between a seller and a buyer. The computer program product may be distributed in the form of a machine-readable storage medium (e.g., compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as memory of the manufacturer's server, a server of the application store, or a relay server.

According to embodiments, each component (e.g., a module or a program) of the above-described components may include a single entity or multiple entities, and some of the multiple entities may be separately disposed in different components. According to embodiments, one or more of the above-described components may be omitted, or one or more other components may be added. Alternatively or additionally, a plurality of components (e.g., modules or programs) may be integrated into a single component. In such a case, according to embodiments, the integrated component may still perform one or more functions of each of the plurality of components in the same or similar manner as they are performed by a corresponding one of the plurality of components before the integration. According to embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device 210 comprising:
a communication module 730 configured to receive sensor data comprising motion information of a user who wears a wearable device 100 from the wearable device 100;
a processor 710 configured to estimate an exercise ability of the user based on the sensor data and generate exercise ability information on the estimated exercise ability; and
a display module 740 configured to output the exercise ability information, wherein the sensor data comprises:
exercise information on a motion performed by the user according to at least one exercise program provided to the user when the wearable device 100 operates in an exercise ability assessment mode, and
the processor 710 is further configured to:
determine the exercise ability information based on the exercise information and
an assessment reference information corresponding to the at least one program.

2. The electronic device of claim 1,
wherein the exercise ability information comprises:
information about at least one of flexibility, flexibility symmetry, physical strength, strength symmetry, core stability, and cardiorespiratory endurance.

3. The electronic device of claim 1 or 2,
wherein the processor 710 is further configured to:
based on the sensor data, assess an exercise posture of the user for a reference exercise posture defined in the at least one exercise program and the exercise ability information based on a result of the assessment.

4. The electronic device of claim 3,
wherein the reference exercise posture comprises a plurality of posture items, and
the processor 710 is further configured to:
based on the sensor data, determine an assessment result of each of the plurality of posture items based on an assessment criterion defined for each of the plurality of posture items.

5. The electronic device of claim 4,
wherein the plurality of posture items comprises:
at least one of a number of motions, motion velocity, a hip joint angle, and body stability during an exercise.

6. The electronic device of claim 5,
wherein the sensor data comprises:
a body motion value of the user obtained by an inertial measurement unit (IMU) 135, 522 comprised in the wearable device 100, and
the processor 710 is further configured to:
assess the body stability of the user based on the body motion value.

7. The electronic device of one of claims 1 to 6,
wherein the wearable device 100 is configured to:
control a driving module 35, 45, 530 of the wearable device 100 to change a magnitude of a resistance force applied to the user when a defined condition is satisfied while the user moves according to the exercise ability assessment mode.

8. The electronic device of claim 7,
wherein the wearable device 100 is configured to:
increase the magnitude of the resistance force when a preset time has arrived or an exercise session has changed.

9. The electronic device of one of claims 1 to 8, wherein the sensor data comprises:
walking motion information on a motion of the user when the user wears the wearable device 100 and walks, and
the processor 710 is further configured to:
estimate a walking index of the user based on the walking motion information.

10. A wearable device 100 worn on a body of a user, the wearable device 100 comprising:
a support frame 20, 25, 50, 55 configured to support the body of the user when the wearable device 100 is worn on the body of the user;
a sensor module 520 configured to obtain sensor data comprising motion information of the user;
a driving module 35, 45, 530 configured to generate torque applied to a body part of the user through the support frame 20, 25, 50, 55;
a communication module 516 configured to transmit the sensor data to an electronic device 210 and receive a control signal from the electronic device 210; and
a processor 512 configured to control the communication module 516 and the sensor module 520,
wherein the sensor data comprises:
exercise information on a motion performed by the user according to at least one exercise program provided to the user when the wearable device 100 operates in an exercise ability assessment mode, and
the processor 512 is further configured to:
control the communication module 516 to transmit the sensor data to the electronic device 210 to cause the electronic device 210 to estimate an exercise ability of the user based on the sensor data, and determine exercise ability information of the user based on the exercise information and assessment
reference information corresponding to the at least one exercise program.

11. The wearable device of claim 10,
wherein the sensor data comprises:
walking motion information on a motion of the user when the user wears the wearable device 100 and walks, and
the processor 512 is further configured to:
control the communication module 516 to transmit the sensor data to the electronic device 210 to cause the electronic device 210 to estimate a walking index of the user based on the walking motion information.

12. The wearable of claim 10 or 11,
wherein the processor 512 is further configured to:
control the driving module 35, 45, 530 to change a magnitude of a resistance force applied to the user when a defined condition is satisfied while the user moves according to the exercise ability assessment mode.

13. The wearable device of one of claims 10 to 12,
wherein the sensor module 520 comprises:
an angle sensor 125, 520, 520-1 and an inertial measurement unit (IMU) 135, 522,
the sensor data comprises
information on a hip joint angle value of the user obtained by the angle sensor 125, 520, 520-1 and information on acceleration and angular velocity according to a motion of the user obtained by the IMU 135, 522, and
the processor 512 is further configured to:
control the communication module 516 to transmit the sensor data to the electronic device 210 to cause the electronic device 210 to estimate an exercise posture of the user for a reference exercise posture defined by the at least one exercise program based on the sensor data and determine the exercise ability information based on a result of the assessment.

14. A method of operating an electronic device 210, the method comprising:
receiving a user input to measure exercise ability information of a user;
in response to the user input, transmitting a control signal to measure the exercise ability information to the wearable device 100;
in response to the transmission of the control signal, when the wearable device 100 operates in an exercise ability assessment mode, receiving, from the wearable device 100, sensor data comprising exercise information on a motion performed by the user according to at least one exercise program provided to the user; and
determining the exercise ability information of the user based on the sensor data,
wherein the determining of the exercise ability information comprises:
determining the exercise ability information based on the exercise information and assessment reference information corresponding to the at least one exercise program.

15. A non-transitory computer-readable storage medium storing instructions that, when executed by a processor, cause the processor to perform the method of claim 14.
